# EUROPEAN PATENT SPECIFICATION

(11) **EP 0 948 604 B1**
(45) Date of publication and mention of the grant of the patent: **21.07.2004**
(21) Application number: 97952333.9
(22) Date of filing: 09.12.1997
(51) Int. Cl.: C12N 9/12

(54) **Screening methods for compounds binding to the Pyk2 polypeptide**
Identifizierungsverfahren für Substanzen die an das Pyk2 Polypeptid binden
Méthodes de criblage de composants capables de lier le polypeptide Pyk2

(30) Priority: 11.12.1996 US 32824 P
(43) Date of publication of application: 13.10.1999
(73) Proprietor: Sugen, Inc., South San Francisco, CA 94080 (US); NEW YORK UNIVERSITY MEDICAL CENTER, New York, NY 10016 (US)
(72) Inventor: LEV, Simma, San Carlos, CA 94070 (US); SCHLESSINGER, Joseph, New York, NY 10011 (US)
(74) Representative: Harrison Goddard Foote
(86) International application number: PCT/US1997/022565
(87) International publication number: WO 1998/026054

(56) References cited:
- WO-A-96/18738
- WO-A-96/40116
- H. SASAKI ET AL.: "Cloning of a cDNA for the human adhesion kinase beta" TUMOR RESEARCH, vol. 30, 1995, pages 37-46, XP002068185
- S. AVRAHAM ET AL.: "Identification and characterization of a novel related adhesion focal tyrosine kinase (RAFTK) from megakaryocytes and brain" JOURNAL OF BIOLOGCAL CHEMISTRY, vol. 270, no. 46, 1995, pages 27742-27751, XP002068186
- S. LEV ET AL.: "Protein tyrosine kinase PYK2 involved in Ca-induced regulation of ion channel an MAP kinase functions" NATURE, vol. 376, 1995, pages 737-745, XP002068187
- H. HERZOG ET AL.: "Molecular cloning and assignement of FK2, a novel human adhesion mkinase to 8p11.2-p22 by nonisotopic in situ hybridization " GENOMICS, vol. 32, 1996, pages 484-486, XP002068188
- H YU ET AL.: "Activation of a novel calcium dependent protein tyrosine kinase" JOURNAL OF BIOLOGICAL CHEMISTRY, vol. 271, no. 47, 1996, pages 29993-29998, XP002068189
- G TOKIWA ET AL.: "Activation of PYK2 y stress signals and coupling with JNK signaling pathway" SCIENCE, vol. 273, 1996, pages 792-794, XP002068190
- C. ZHENG ET AL.: "Interaction of cellular proteins with a novel tyrosine kinase PY2" MOLECULAR BIOLOGY OF THE CELL, vol. 7 (suppl), 1996, page 360A XP002068191
- . DIKIC ET AL.: "Arole for PYK2 and Src in linking G-rotein-coupled receptors with MAP kinase activation" NATURE, vol. 383, 1996, pages 547-550, XP002068192

## Description

### Related Applications

The present application is related to U.S. application Serial No. 08/460,626, filed June 2, 1995, which is a continuation-in-part application of U.S. patent application Serial No. 08/357,642, filed December 15, 1994.

### Introduction

The present invention relates generally to a novel protein termed PYK2 and related products and methods.

### Background of the Invention

None of the following discussion of the background of the invention is admitted to be prior art to the invention.

Cellular signal transduction is a fundamental mechanism whereby external stimuli that regulate diverse cellular processes are relayed to the interior of cells. One of the key biochemical mechanisms of signal transduction involves the reversible phosphorylation of tyrosine residues on proteins. The phosphorylation state of a protein is modified through the reciprocal actions of tyrosine phosphatases (TPs) and tyrosine kinases (TKs), including receptor tyrosine kinases and non-receptor tyrosine kinases.

Receptor tyrosine kinases (RTKs) belong to a family of transmembrane proteins and have been implicated in cellular signaling pathways. The predominant biological activity of some RTKs is the stimulation of cell growth and proliferation, while other RTKs are involved in arresting growth and promoting differentiation. In some instances, a single tyrosine kinase can inhibit, or stimulate, cell proliferation depending on the cellular environment in which it is expressed.

RTKs are composed of at least three domains: an extracellular ligand binding domain, a transmembrane domain and a cytoplasmic catalytic domain that can phosphorylate tyrosine residues. Ligand binding to membrane-bound receptors induces the formation of receptor dimers and allosteric changes that activate the intracellular kinase domains and result in the self phosphorylation (autophosphorylation and/or transphosphorylation) of the receptor on tyrosine residues. Individual phosphotyrosine residues of the cytoplasmic domains of receptors may serve as specific binding sites that interact with a host of cytoplasmic signaling molecules, thereby activating various signal transduction pathways.

The intracellular, cytoplasmic, non-receptor protein tyrosine kinases do not contain a hydrophobic trans membrane domain or an extracellular domain and share non-catalytic domains in addition to sharing their catalytic kinase domains. Such non-catalytic domains include the SH2 domains and SH3 domains. The non-catalytic domains are thought to be important in the regulation of protein-protein interacions during signal transduction.

Focal adhesion kinase (FAK) is a cytoplasmic protein tyrosine kinase that is localized to focal adhesions. Schaller, et al., Proc. Natl. Acad. Sci. U.S.A., 89:5192-5196 (1992); Cobb et al., Molecular and Cellular Biology, 14 (1) :147-155 (1994). In some cells the C-terminal domain of FAK is expressed autonomously as a 41 kDa protein called FRNK and the 140 C-terminal residues of FAK contain a focal adhesion targeting (FAT)domain. The cDNA's encoding FRNK are given in Schaller et al., Molecular and Cellular Biology, 13(2):785-791 (1993). The FAT domain was identified and said to be required for localization of FAK to cellular focal adhesions in Hilderbrand et al., The Journal of Cell Biology, 123(4) :993-1005 (1993).

A central feature of signal transduction is the reversible phosphorylation of certain proteins. Receptor phosphorylation stimulates a physical association of the activated receptor with target molecules, which either are or are not phosphorylated. Some of the target molecules such as phospholipase Cy are in turn phosphorylated and activated. Such phosphorylation transmits a signal to the cytoplasm. Other target molecules are not phosphorylated, but assist in signal transmission by acting as adapter molecules for secondary signal transducer proteins. For example, receptor phosphorylation and the subsequent allosteric changes in the receptor recruit the Grb-2/SOS complex to the catalytic domain of the receptor where its proximity to the membrane allows it to activate ras. The secondary signal transducer molecules generated by activated receptors result in a signal cascade that regulates cell functions such as cell division or differentiation. Reviews describing intracellular signal transduction include Aaronson, Science, 254:1146-1153, 1991; Schlessinger, Trends Biochem. Sci., 13:443-447, 1988; and Ullrich and Schlessinger, Cell, 61:203-212, 1990.

Several protein tyrosine kinases are highly expressed in the central nervous system and there is evidence that protein phosphorylation plays a crucial regulatory role in the nervous system. Neurotrophic factors that control the differentiation and maintain the survival of different types of neuronal cells mediate their biological effects by binding to and activating cell surface receptors with intrinsic protein tyrosine kinase activity. Furthermore, protein phosphorylation is a key regulatory mechanism of membrane excitability and ion channel function.

Tyrosine phosphorylation regulates the function of several ion-channels in the central nervous system. Protein kinase C (PKC) can regulate the action of a variety of ion channels including voltage-gated potassium channels, voltage dependent sodium channels as well as the nicotinic acetycholine receptor. The action of the NMDA receptor can be modulated by protein-tyrosine kinases and phosphatases. Moreover, tyrosine phosphorylation of the nicotine acetylcholine receptors (AchR) increases its rate of desensitization, and may play role in regulation of AchR distribution on the cell membrane. Another example is the delayed rectifier-type K+ channel, termed Kv1.2 also called RAK, RBKZ, RCK5 and NGKI). This channel is highly expressed in the brain and cardiac atria, and can be regulated by tyrosine phosphorylation. Tyrosine phosphorylation of Kv1.2 is associated with suppression of Kv1.2- currents. Suppression of Kv1.2 currents was induced bv a variety of stimuli including carbachol, bradykinin, PMA and calcium ionophore.

The Ras/MAP kinase signal transduction pathway is highly conserved in evolution and plays an important role in the control of cell growth and differentiation. The MAP kinase signalling pathway in PC12 cells can be activated by NGF, by peptide hormones that activate G-protein coupled receptors, by phorbol ester as well as by calcium influx following membrane depolarization. However, the mechanism underlying activation of the Ras/MAP kinase signaling pathway by G-protein coupled receptors as well as by calcium influx are not known. Shc is involved in the coupling of both receptor and non-receptor tyrosine kinases to the Ras/MAPK signaling pathways. Overexpression of Shc leads to transformation of 3T3 cells and to neuronal differentiation of pC12 cells. Moreover, Shc induced differentiation of PC12 cells is blocked by a dominant mutant of Ras indicating that Shc acts upstream of Ras. Tyrosine phosphorylated Shc can activate the Ras signaling pathways by binding to the SH2 domain of the adaptor protein Grb2 that is complexed to the guanine nucleotide releasing factor Sos via its SH3 domains.

Signal transduction pathways that regulate ion channels (e.g., potassium channels and calcium channels) involve G proteins which function as intermediaries between receptors and effectors. Gilman, Ann. Rev. Biochem., 56:615-649 (1987); Brown and Birnbaumer, Ann. Rev. Physiol., 52:197-213 (1990). G-coupled protein receptors are receptors for neurotransmitters, ligands that are responsible for signal production in nerve cells as well as for regulation of proliferation and differentiation of nerves and other cell types. Neurotransmitter receptors exist as different subtypes which are differentially expressed in various tissues and neurotransmitters such as acetylcholine evoke responses throughout the central and peripheral nervous systems. The muscarinic acetylcholine receptors play important roles in a variety of complex neural activities such as learning, memory, arousal and motor and sensory modulation. These receptors have also been implicated in several central nervous system disorders such as Alzheimer's disease, Parkinson's disease, depression and schizophrenia.

Some agents that are involved in a signal transduction pathway regulating one ion channel, for example a potassium channel, may also be involved in one or more other pathways regulating one or more other ion channels,for example a calcium channel. Dolphin, Ann. Rev.Physiol., 52:243-55 (1990); Wilk-Blaszczak et al., Neuron, 12:109-116 (1994). Ion channels may be regulated either with or without a cytosolic second messenger. Hille,Neuron, 9: 187-195 (1992). One possible cytosolic second messenger is a tyrosine kinase. Huang et al., Cell,75:1145-1156 (1993).

The receptors involved in the signal transduction pathways that, regulate ion channels are ultimately linked to the ion channels by various intermediate,events and agents. For example, such events include an increase in intracellular calcium and inositol triphosphate and production of endothelin. Frucht, et al., Cancer Research, 5 2:1114-1122(1992) ; Schrey, et al. , CancerResearch,52:1786-1790 (1992). Intermediary agents include bombesin, which stimulates DNA synthesis and the phosphorylation of a specific protein kinase C substrate. Rodriguez-Pena, et al., Biochemical and Biophysical Research Communication, 140(1) :379-385 (1986) ; Fisher and Schonbrunn, The Journal of Biological Chemistry, 263 (6) :2208-2816 (1988).

### Summary of the Invention

PYK2 polypeptides are involved in various signal transduction pathways and thus the present invention provides several agents and methods useful for diagnosing, treating, and preventing various diseases or conditions associated with abnormalities in these pathways.

The present invention is based in part upon the identification and isolation of a novel non-receptor tyrosine kinase, termed PYK2. Without wishing to be bound to any particular theory, it appears that PYK2 participates in at least two signal transduction pathways. The first signal transduction pathway is activated when such extracellular signals as bradykinin or acetyl-choline bind G protein-coupled receptor protein kinases. Receptor protein kinases that are G protein coupled include, but are not limited to, Lyn and Syk which are located in the B-cells of the immune system.

Another PYK2 signal transduction pathway is activiated when growth factors bind receptor protein kinases. The receptors dimerize and then cross phosphorylate one another. The phosphate moieties now attached to the receptor protein kinases attract other signaling molecules to these receptors located at the plasma membrane. Such signalling molecules can be, among others, *sos, shc,* or *grb* . Complexes such as an EGFR/*grb*-2/sos complex can activate *ras* molecules also located at the plasma membrane. *Ras* molecules can then activate signalling molecules that are not attached to the plasma membrane. These cytsolic signalling molecules can propogate an extracellular signal to the nucleus and promote the production of cellular agents necessary for a response to the extracellular signal. Examples of the cytosolic signalling molecules are proteins involved in the MAP kinase signalling cascade.

The description provided herein indicates that PYK2 may combine the G protein-coupled pathway with the *sos*/*grb* pathway for MAP kinase signal transduction activation in repsonse to stimulation by G protein-coupled receptors. The invention also indicates that PYK2 brings these two pathways together by binding src, another protein kinase involved in signal transduction events. Thus, the invention provides new targets for therapeutics effective for treating cell proliferative diseases such as cancer and/or cell differentiation disorders.

PYK2 has a predicted molecular weight of 111 kD and contains five domains: (1) a relatively long N-terminal domain from amino acid 1 to amino acid 417; ( 2 ) a kinase catalytic domain from amino acid 418 to amino acid 679 contains nucleotide binding domain at amino acid 431 to amino acid 439 and an ATP binding site at amino acid 457); a proline rich domain from amino acid 713 to amino acid 733; (4) another proline rich domain from amino acid 843 to amino acid 860; and (5) a C-terminal focal adhesion targeting (FAT) domain from amino acid 861 to amino acid 1009. PYK2 does not contain a SH2 or SH3 domain. Amino acids 696-1009 of PYK2 show homology to FRWK, the c-terminal fragments of FAK. Other features of the PYK2 sequence include the following: (1) amino acid 402 is the major autophosphorylation site of PYK2 and is a Src SH2 binding site; (2) amino acid 599 is an autophosphorylation site in an activation loop of PYK2 kinase; (3) amino acid 881 is an autophosphorylation site and a GRB2 binding site; and (4) amino acid 906 is an auto phosphorylation site and SHP-2 (PTP-10) binding site.

The FAT domain of PYK2 has about 62% similarity to the FAT domain of another non-receptor tyrosine kinase, FAK, which is also activated by G-coupled proteins. The overall similarity between PYKZ and FAK is about 52 %. PYK2 is expressed principally in neural tissues, although expression can also be detected in hematopoietic cells at early stages of development and in some tumor cell lines. The expression of PYK2 does not correspond with the expression of FAK.

PYK2 is believed to regulate the activity of potassium channels in response to neurotransmitter signalling. PYKz enzymatic activity is positively regulated by phosphorylation on tyrosine and results in response to binding of bradykinin, TPA, calcium ionophore, carbachol, TPA + forskolin, and membrane depolarization. The combination of toxins known to positively regulate G-coupled receptor signalling (such as pertusis toxin, cholera toxins, TPA and bradykinin) increases the phosphorylation of PYK2.

Activated PYK2 phosphorylates RAK, a delayed rectfier type potassium channel, and thus suppresses RAK activity. In the same system, FAK does not phosphorylate RAK. PYK2 is responsible for regulating neurotransmitter signalling and thus may be used to treat conditions of nervous system by enhancing or inhibiting such signalling.

One aspect of the invention features a method of detecting the presence or amount of a compound capable of binding to a PYK2 polypeptide, wherein said compound is an indolinone. The method involves incubating the compound with a PYK2 polypeptide and detecting the presence or amount of the compound bound to the PYK2 polypeptide.

In preferred embodiments, the compound inhibits a phosphorylation activity of the PYK2 polypeptide.

In another aspect the invention features a method of screening potential agents useful for treatment of a disease or condition characterized by an abnormality in a signal transduction pathway that contains an interaction between a PYK2 polypeptide and a natural binding partner (NBP). The method involves assaying potential agents for those able to promote or disrupt the interaction as an indication of a useful agent. The natural binding partner is Src and the pathway involves a G protein-coupled receptor but does not involve a growth factor receptor, and the potential agents are one or more indolinones.

Preferably the indolinone is an oxindolinone.

Specific diseases or disorders which might be treated or prevented, based upon the affected cells include: myasthenia gravis; neuroblastoma; disorders caused by neuronal toxins such as cholera toxin, pertusis toxin, or snakevenom; acute megakaryocytic myelosis; thrombocytopenia; those of the central nervous system such as seizures, stroke, head trauma, spinal cord injury, hypoxia-induced nerve cell damage such as in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, and Tourette's syndrome. Conditions that may be treated by PYK2 inhibitors include epilepsy, schizophrenia, extreme hyperactivity in children, chronic pain, and acute pain. Examples of conditions that may be treated by PYK2 enhancers (for example a phosphatase inhibitor) include stroke, Alzheimer's, Parkinson's, other neurodegenerative diseases and migraine. Preferred disorders include epilepsy, stroke, schizophrenia, and Parkinson's disorder, as there is a well established relationship between these disorders and the function of potassium channels.

The invention also provides a method of screening for modulators of PYK2 signalling pathways comprising the steps of: mixing two or more components selected from the group consisting of PYK2, Src, bradykinin, LPA, and one or more of said modulators, wherein the modulators are one or more indolinones; and monitoring the effect of the modulators on the PYK2 signalling pathways.

Preferably the indolinone is an oxindolinone. Preferably the method involves screening agents capable of promoting or disrupting the binding of the SH2 domain of Src to the tyrosine at position 402 of PYK2.

Preferably the effect is a phosphorylation state of PYK2 or Src.

Other features and advantages of the invention will be apparent from the following description of the preferred embodiments thereof, and from the claims.

### Brief Description of the Figures and Tables

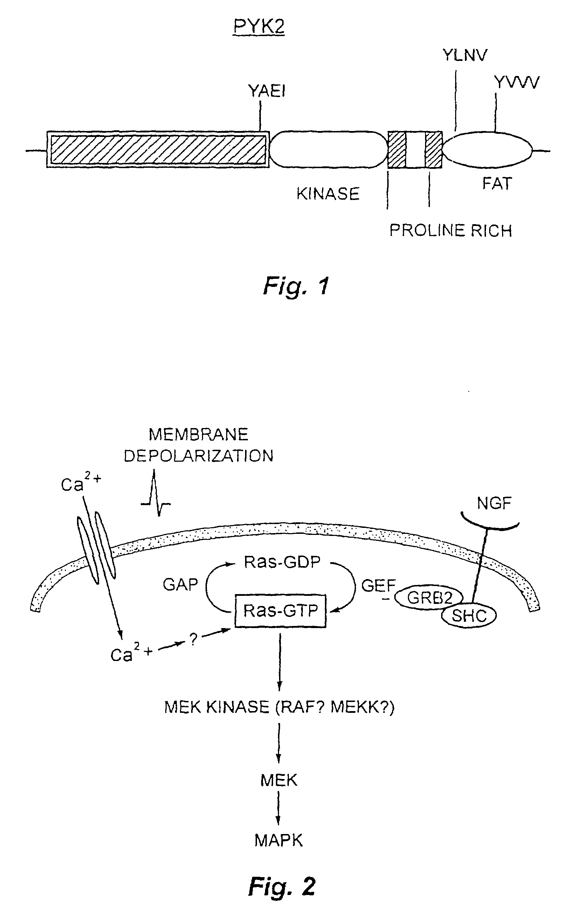

Figure 1 shows a schematic representation of the PYK2 domains (including a kinase domain, a proline rich domain, and a Fat domain) and potential binding sites (including YAEI, YLNV, and YVVV).
Figure 2 shows a possible mechanism for the membrane depolarization and calcium influx that stimulate MEK and MAP kinase via activation 'of Ras. In PC12 cells, membrane depolarization leads to calcium influx through L-type calcium channels and activates MAP kinase. Calcium influx leads to activation of Ras and the activation of MAP in response to calcium influx is inhibited by a dominant negative mutant of Ras. Elevation of intracellular calcium concentration by various stimuli leads to the activation of PYK2. PYK2 recruits Shc/Grb2/Sos complex leading to the activation of a signaling pathway composed of Ras, Raf, MAPKK, MAPK to relay signals to the cell nucleus.

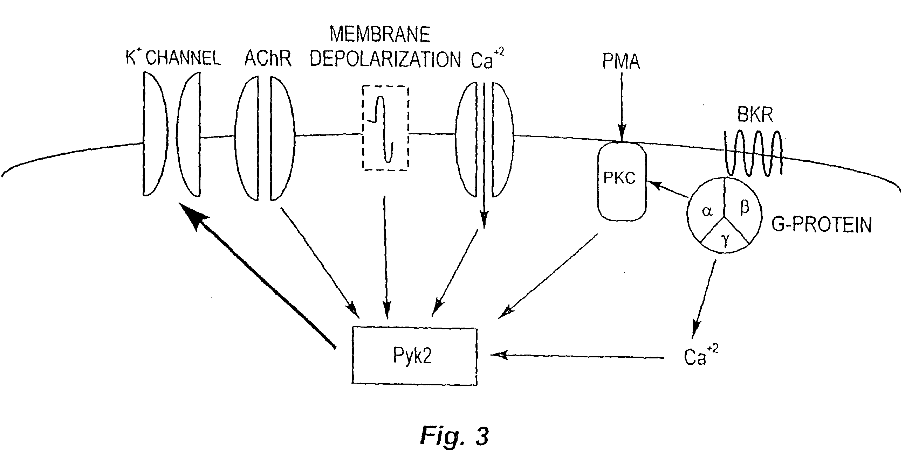

Figure 3 shows a model for the extracellular stimuli that activate PYK2 and potential target molecule that is tyrosine phosphorylated in response to PYK2 activation. The tyrosine kinase activity of PYK2 is activated by a variety of extracellular signals that stimulate calcium influx including activation of the nicotnic acetylcholine receptor by carbachol, membrane depolarization by KCl (75mM), and treatment with a calcium ionophore. Activation of PYK2 by these stimuli requires the presence of extracellular calcium. PYK2 is also stimulated in response to bradykinin (BK) induced activation of its G-protein coupled receptor leading to PI hydrolysis and Ca+2 release from internal stores. PYK2 is also activated in response to phorbol ester (PMA) treatment that binds to and activates several PKC isozymes. Co-expression experiments in transfected cells and in frog oocytes show that activation of PYK2 leads to tyrosine phosphorylation (thick arrow) of the delayed rectifier-type K+ channel Kv1.2 and to suppression of Kv1.2 channel mediated currents.

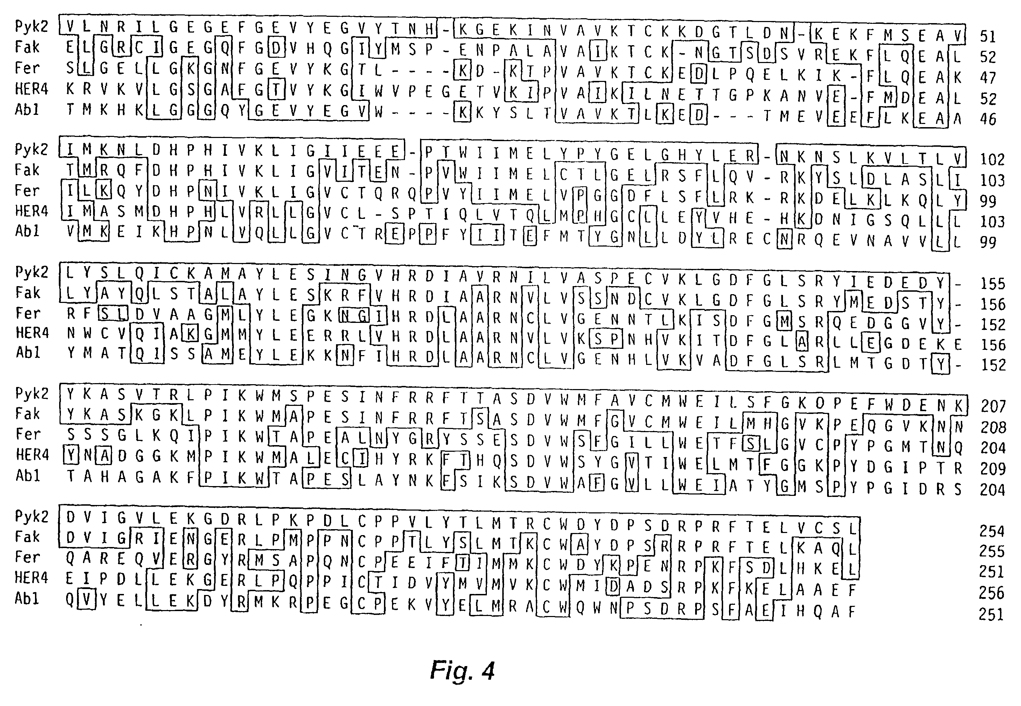

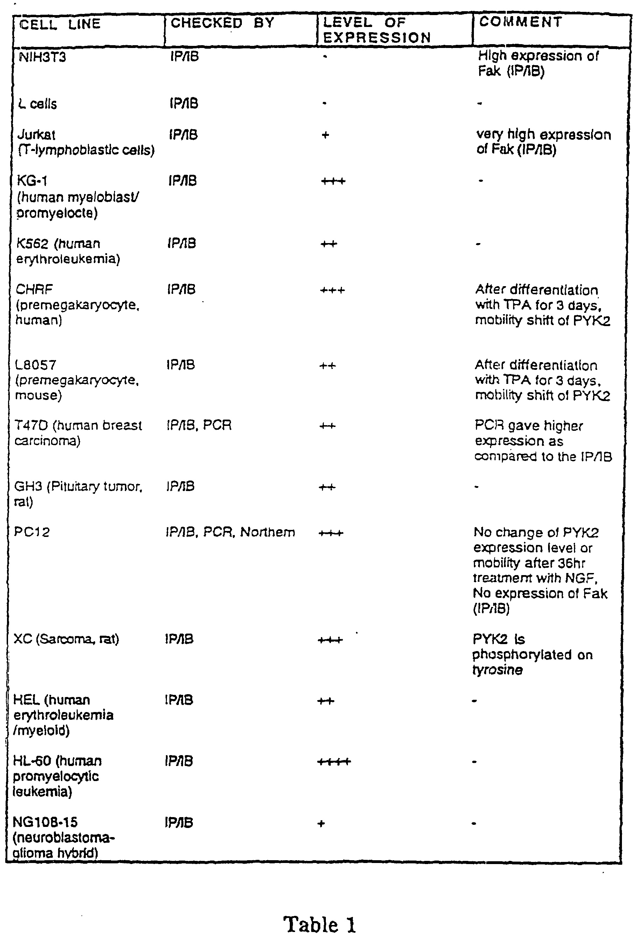

Figure 4 shows an alignment of PYK-2 amino acids to those of 4 other proteins, Fak, Fer, HER4 and AB1. Table 1 shows the expression pattern and levels of PYK2 in various cell lines as checked by multiple methods.

### Description of the Preferred Embodiments

The present invention relates to assays utilizing PYK2 polypeptides. Those skilled in the art will recognize that many of the methods described below in relation to PYK-2, a NBP, or a complex of PYK-2 and a NBP could also be utilized with respect to the other members of this group.

We describe the isolation and characterization of a novel non-receptor tyrosine kinase termed PYK2 , that is highly expressed in the nervous system and in the adult rat brain. PYK2 is a second member of Fak family of non-receptor protein tyrosine kinases. However, PYK2 exhibits diffuse cytoplasmic localization unlike the preferential localization of Fak in focal adhesion areas.

The examples presented herein reveal a novel mechanism for the coupling, between G-protein coupled receptors and the MAP kinase signaling pathway. We also show that calcium influx induced by membrane depolorization following activation of the nicotinic acetylcholine receptor or other stimuli that cause calcium influx lead to the activation of PYK2, tyrosine phosphorylation of Shc, recruitment of Grb2/Sos and activation of the MAP kinase signaling pathway.

PYK2 is activated by extracellular signals that lead to calcium influx or calcium release from internal stores. PYK2 is phosphorylated on tyrosine residues in response to a variety of external stimuli that cause membrane depolarization and Ca ⁺² influx such as the activation of the nicotinic acetylcholine receptor. Tyrosine phosphorylation of PYK2 is also stimulated by the neuropeptide bradykinin that activates a G-protein coupled receptor as well as by phorbol myristate acetate (PMA). Experiments in transfected cells and in Xenopus oocytes, microinjected with PYK2 mRNA, indicate that activation of PYK2 can lead to tyrosine phosphorylation of a delayed rectifier-type potassium channel protein and to suppression of potassium currents via this channel. These results suggest a novel mechanism by which a non-receptor tyrosine kinases, in the nervous system, can be both activated by and can modulate the action of ion-channel proteins. Activation of PYK2 in PC12 cells by the same stimuli leads to the recruitment of Shc/Grb2/Sos complex and to the activation of the MAP kinase signaling pathway that relays signals to the cell nucleus. The experiments presented thus show that PYK2 may also provide a link between G protein coupled receptors and calcium influx and the MAP kinase signaling pathway; a pathway that relays signals from the cell surface to regulate transcriptional events in the nucleus. Overexpression of PYK2 leads to activation of MAP kinase. Moreover, the effects of PYK2 on tyrosine phosphorylation and action of the Kv1.2 potassium channel reveals a novel mechanism for heterologous regulation of ion-channel function by activation of an intermediate protein tyrosine kinase. PYK2 can, therefore, couple neuropeptide hormones that act via G-protein coupled receptors that stimulate phosphotydinositot hydrolysis and the action of target channel molecules.

Transient co-expression experiments of PYK2 with the delayed rectifier K+ channel Kv1.2 show that the channel protein undergoes tyrosine phosphorylation in response to PYK2 activation. Moreover, currents exhibited by Kv1.2 channel expressed in frog oocytes were blocked by co-expression of the PYK2 protein. However, co-expression of a kinase negative mutant of PYK2 released PMA induced suppression of Kv1.2 currents. PYK2 activation may provide a rapid and highly localized control mechanism for ion channel function and kinase activation induced by neuronal stimuli that elevate intracellular calcium leading, to neuronal integration and synaptic efficacy.

These results reveal a role for PYK2 in activation of the MAP kinase signaling pathway by ion channels, calcium influx and G-protein coupled receptors in PC12 cells and may provide a mechanism for signal transduction induced by these stimuli in the nervous system. Furthermore, tyrosine phosphorylation of Shc in response to membrane depolarization and carbachol treatment was dependent on the presence of extracellular calcium, indicating that calcium-influx plays a role in regulation of Shc phosphorylation by these stimuli. Similarly, PYK2 may modulate the action of ion channels that mediate their responses and are sensitive to intracellular calcium concentration. PYK2 may therefore provide an autoregulatory role for the very same channel responsible for PYK2 activation. A potential target of PYK2 is the nicotinic acetylcholine receptor. Activation of the nicotinic acetylcholine receptor in PC12 cells leads to strong and rapid tyrosine phosphorylation of PYK2. The nicotinic acetylcholine receptor is subject to gylation and can modulate the activity of the tyrosine phosphorylation. Tyrosine phosphorylation of Shc in response to carbachol treatment is induced via stimulation of the nicotinic acetylcholive receptor as determined by pharmacological ]analysis. The nicotinic agonist DMPP induced phosphorylation of Shc, whereas muscarine had no effect, the nicotinic antagonist mecamylamine blocked the effect of carbachol, whereas the muscarinic antagonist atropine had no effect. The effect of carbachol on tyrosine phosphoryiation of Shc was transient with maximum tyrosine phosphorylation detected after one minute followed by a rapid decline. NGF however, induced persistent stimulation of Shc phosphorylation for as long as five hours after the addition of NGF. The duration of Shc phosphorylation may have an important impact on the Ras signaling pathway and gene expression induced by these stimuli.

The model presented herein may represent the mechanism underlying calcium mediated regulation of gene expression in neuronal cells induced by MMDA receptor or voltage sensitive calcium channels. The expression pattern of PYK2, the external stimuli that activate this kinase together with its role in the control of MAP kinase signaling pathway suggests a potential role for PYK2 in the control of a broad array of processes in the central nervous system including neuronal plasticity in the nervous system.

Since PYK2 activity is regulated by intracellular calcium level, both the temporal and spatial pattern of PYK2 activation may represent a carbon copy or a replica of the spatial and temporal profile of intracellular calcium concentration. Calcium concentration inside cells is highly localized because of a variety of calcium binding proteins that provide a strong buffer. Moreover, in excitable cells the level of calcium can be regulated by voltage dependent calcium channels that induce large and transient increase in intracellular calcium concentration leading to calcium oscilations and calcium waves. PYK2 may provide a mechanism for rapid and highly localized control of ion channel function, as well as, localized activation of the MAP kinase signaling pathway. Preliminary immunolocalization analysis indicates that PYK2 is expressed in hippocampal postsynaptic dendritic spines, suggesting a potential role of this kinase in synaptic plasticity mediated by calcium influx. Potassium channels are frequent targets for phosphorylation by tyrosine kinases that are activated by neurotransmitters or neuropeptides. Phosphorylation of other voltage gated channels or neurotransmitter receptors provides an important regulatory mechanism for modulation. Thus, PYK2 may represent an important coupling molecule between neuropeptides that activate G-protein coupled receptors or neurotransmitters that stimulate Ca+2 influx and downstream signaling events that reculate neuronal plasticity, cell excitability, and synaptic efficacy. We have demonstrated that PYK2 is rapidly activated in response to a wide variety of extracellular stimuli. These stimuli include activation of an ion channel, stimulation of a G-protein coupled receptor, calcium influx following membrane depolarization as well as phorbol ester stimulation. Although the molecular mechanisms by which these signals induce the activation of PYK2 are not yet known, our results clearly show that elevation of intracellular calcium concentrations is crucial for PYK2 activation. The effect of PMA on PYK2 activation may indicate that PYK2 can be also activated by a PKC dependent pathway. The fact that PYK2 can be activated by an ion-channel, such as the nicotinic acetylcholine receptor, and by intracellular calcium raised the possibility that PYK2 may regulate ion-channel function by tyrosine phosphorylation.

We further analyzed agonist-induced MAP kinase activity in PC12 cell lines which stably overexpress a dominant interfering mutant of Grb2 lacking the N-terminal SH3 domain (Grb2 DN-SH3) or in PC12 cells which stably 15 overexpress the proline rich tail of Sos (Sos-CT). Xie *et al., J. Biol. Chem*. 270, 30717-30724 (1995); Gishizky *et al*., *Proc. Natl. Acad. Sci . USA 92*, 10889-10893 (1995). Overexpression of Grb2' DN-SH3 in PC12 cells completely blocked LPA- or bradykinin-induced MAP kinase activation. Overexpression of Sos-CT strongly reduced MAP kinase activation in response to LPA and bradykinin stimulation. However, activation of PYK2 or Src was not affected by the dominant interfering mutants of Grb2 and Sos confirming that PYK2 and Src act upstream of Grb2 and *Sos* in the cascade of events leading to MAP kinase activation. Experiments presented herein demonstrate that PYK2 can link both Gi- or Gq-protein coupled receptors with the MAP kinase signaling pathway in PC12 cells. Phosphorylation on Tyr402 of PYK2 leads to binding of the SH2 domain of Src and subsequent Src activation in response to either Gi- or Gq-protein coupled receptors. Overexpression of activated Src (Y527F) in PC12 cells induces tyrosine phosphorylation of PYK2, but does not stimulate PYK2 kinase activity.

It is possible therefore, that tyrosine phosphorylation of PYK2 is in part mediated by Src, thus generating docking sites for additional signaling proteins that are recruited by PYK2. We have demonstrated that activation of PYK2 leads to both direct recruitment of Grb2/Sos as well as indirect recruitment via tyrosine phosphorylation of Shc. We present experiments demonstrating that dominant interfering mutants of Grb2 or Sos confer strong inhibition on LPA- or bradykinin-induced activation of MAP kinase. These results are in accord with recent studies demonstrating that a Sos deletion mutant or a dominant interfering mutant of Shc blocked LPA- or thrombin-induced activation of MAP kinase respectively van Biesen *et al., Nature* 376:781-784 (1995); Chen *et al., EMBO J.* 15:1037-1044 (1996).

Taken together, these experiments underscore the central role of the Shc/Grb2/Sos complex in mediating MAP kinase activation not only by receptor tyrosine kinases, but also by Gi- and Gq-protein coupled receptors. Wan *et al*., Nature 380:541-544 (1996). In avian B-cells both Lyn and Syk are essential for activation of MAP kinase by Gi- and Gq-protein coupled receptors. It appears therefore that a combination of different protein tyrosine kinases in different tissues and cell types may link G-protein coupled receptor with the MAP kinase signaling pathway. Src family protein tyrosine kinases, which are expressed in every cell type and tissue, appear to be a common and important component of this pathway, by acting together with cell-type specific protein tyrosine kinases such as PYK2 in PC12 cells or Syk in avian B-cells to bring about a cell-type specific signal for linking G-protein coupled 30 receptors with MAP kinase signaling pathway and hence the transcriptional machinery.

### Examples

The examples below are non-limiting and are merely representative of various aspects and features of the procedures used to identify the full-length nucleic and amino acid sequences of PYK-2. Experiments demonstrating PYK-2 expression, interaction and signalling activities are also provided.

### Materials and Methods

### Chemicals

Bradykinin, pertusis toxin, cholera toxin, forskolin, phorbol 12-myristate 13-acetate (PMA), calcium ionophore A23187, carbachol, muscarine, atrophine, mecamylamine, and 1,1-dimethyl-4-phenyl piperazinium iodide (DMPP) were purchased from Sigma.

### Cloning of PYK2 cDNA

We have used the Grb2 adaptor protein as a specific probe for screening-expression libraries in order to isolate Grb2 binding proteins. One of the cloned proteins encoded a protein tyrosine kinase that contains a proline rich region that can bind in vitro to the SH3 domains of Grb2. This protein was termed PYK1 for proline rich tyrosine kinase 1. Comparison of the amino acid sequence of PYK1 to other tyrosine kinases, indicated that PYK1 is related to the Ack protein tyrosine kinase. Analysis of PYK1 sequence indicated that this kinase represents a new class of cytoplasmic protein tyrosine kinases. In an attempt to isolate kinases related to PYK1, we applied the polymerase chain reaction (PCR) utilizing degenerate oligonucleotide primers, derived from PYK1 sequence according to the conserved motifs of the catalytic domains of PTKs. RNA from rat spinal cord was used to prepare cDNA utilizing the reverse transcriptase of Molony murine leukemia virus (^{BRL}) according to the manufacturer's protocol. The cDNA was amplified by PCR utilizing degenerate oligonucleotide primers corresponding to conserved tyrosine kinase motifs from subdomains TK6 and TK9 of PYK1; (the sense and antisense primers correspond to amino acid sequences IHRDLAARN [SEQ. ID NO 3] and WMFGVTLW [SEQ. ID NO 41 respectively). The PCR was carried out under the following conditions; 1 min at 94°C; 1 min at 50°C and 1 min at 68°C for 35 cycles. PCR products were electrophoresed, checked by the size (-210bp), purified and subcloned into pBluescript (Stratagene). Novel clones were screened by DNA sequencing. The cDNA insert of clone #38 was used as probe to screen human brain cDNA libraries (human fetal brain λgt 10 and human brain λgt 11, 6x105 recombinant clones each) essentially as described by Maniatis.

The complete amino acid sequence of a novel protein tyrosine kinase was isolated from human brain cDNA library and termed PYK2. The open reading frame of PYK2 encodes a protein of 1009 amino acids containing a long N-terminal sequence of 424 amino acids followed by a protein tyrosine kinase domain, two proline rich domains (29% and 23.3% proline respectively) and a large carboxy terminal region. The kinase domain of PYK2, contains several sequence motifs conserved among protein tyrosine kinases, including the tripeptide motif DFG, found in most kinases, and a consensus ATP binding motif GXGXXG followed by AXK sequence 17 amino acids residues downstream.

Comparison of the amino acid sequence of the kinase domain of PYK2 with other protein tyrosine kinases showed that the kinase core of PYK2 is most similar to the protein tyrosine kinase domains of Fak, Fer, Her4 and Abl. In addition to the sequence homology in the kinase domain, the flanking sequences and the overall structural organization of the PYK2 protein are similar to those of FAK indicating that PYK2 belong to the same family of non-receptor similar to those of Fak protein tyrosine kinases.

### DNA Sequencing and Analysis

DNA sequencing was performed on both strands utilizing series of oligonucleotide primers and subclones. The nucleotide sequence and the deduced amino acid sequence were subjected to homology search with Genbank and PIR databases using FASTA and BLAST mail-server program.

### Northern blot analysis

Total RNA was isolated from mouse tissues by the acid guanidinium thicynate-phenol-chloroform method (Anal. Biochem. 162; 156, 1987). Poly (A)⁺ RNA was denaturated with formaldehyde and electrophoresed on a 1% agarose/0.7% formaldehyde gel. RNAs were transferred to a nitro-cellulose membrane and hybridized with ³²P-labeled probe that contained the cDNA insert of clone #38 as described above.

### Antibodies

Antibodies against PYK2 were raised in rabbits immunized (HTI) either by GST fusion protein containing residues 362- 647 or PYK2 or by synthetic peptide corresponding to the 15 amino acids at the N-terminal end of PYK2. Antisera were checked by immunoprecipitation and immunoblot analysis, and the specificity was confirmed either by reactivity to the related protein Fak or by competition with the antigenic or control peptides.

Antibodies against PYK-2 were raised in rabbits immunized either with GST fusion protein containing residues of PYK-2 or with synthetic peptide corresponding the 15 amino acids at the N-terminal end of PYK-2. The antibodies are specific to PYK-2 and they do not cross react with FAK.

### Cells and cell culture

PC12-rat pheochromocytoma cells were cultured in Dulbecco's modified Eagle's medium containing 10% horse serum, 5% fetal bovine serum, 100,µg/ml streptomycin and 100 units of penicillin/ml. NIH3T3, 293, GP+E-86 and PA317 cells were grown in Dulbecco's modified Eagle's medium containing 10% fetal bovine serum, 100 µg/ml streptomycin and 100 units of penicillin/ml.

### Transfections and infections

For stable expression in PC12 cells, PYK2 was subcloned into the retroviral vector pLXSN (Miller and Rosman, Biotechniques 7: 980, 1989). The construct was used to transfect GP+E-86 cells using lipofectimine reagent (GIBCO BRL). 48 hours after transfection, virus containing supernatants were collected. Pure retrovirus-containing cell-free supernatant were added to PC12 cells in the presence of polybrene (8, µg/ml, Aldrich) for 4 hours (MCB 12 491 , 1992) . After 24 hours, infected PC12 cells were split into growing medium containing 350 µl/mg G418. G418 resistant colonies were isolated two to three weeks later and the level of expression was determined by western blot analysis.

Stable cell lines of NIH3T3 that overexpress PYK2 were established by cotransfection of PYK2 subcloned into pLSV together with pSV2neo utilizing lipofectamine reagent (GIBCO BRL). Following transfection the cells were grown in Dulbecco's modified Eagle's medium containing 10% fetal bovine serum and lmg/ml G418. Transient transfections into 293 cells were performed by using a calcium phosphate technique.

### Constructs

**GST-PYK2-** a DNA fragment of λ900bp corresponding to residues 362-647 of PYK2 was amplified by PCR utilizing the following oligonucleotide primers:

The PCR product was digested with BamHI and EcoRI and subcloned into pGEX3X (Pharmacia). Expression of GST-PYK2 fusion protein was induced by the 1mM IPTG essentially as described by Smith et al., (Gene 67:31, 1988). The fusion protein was isolated by electroelution from SDS-PAGE.

**PYK2-** The full length cDNA sequence of PYK2 was subcloned into the following mammalian expression vectors: pLSV; downstream the SV40 early promoter, pLXSN-retroviral vector; downstream the Mo-MuLV long terminal repeat; pRK5; downstream the CMV promoter.

**PYK2-HA-** the influenza virus hemagglutinin peptide (YPYDVPDYAS) [SEQ. ID NO 71 was fused to the C-terminal end of PYK2 utilizing the following oligonucleotide primers in the PCR: 5'-CACAATGTCTTCAAACGCCAC-3' [SEQ. ID NO 8] and 5'-GGCTCTAGATCACGATGCGTAGTCAGGGACATCGTATGGGRACTCTGCAGGTGGGTGGGC CAG-3'. [SEQ. ID NO 9]. The amplified fragment was digested with RsrII and Xbal and used to substitute the corresponding fragment of PYK2. The nucleotide sequence of the final construct was confirmed by DNA sequencing.

**Kinase negative mutant-** In order to construct a kinase negative mutant, the Lys at position 457 was substituted to Ala by site directed mutagenesis (Clontech). The oligonucleotide sequence was designed to create a new restriction site of NruI. The nucleotide sequence of the mutant was confirmed by DNA sequencing. The oligonucleotide sequence that was used for mutagenesis is: 5'CAATGTAGCTGTCGCGACCTGCAAGAAAGAC-3' [SEQ. ID NO 10] (Nrul site - bold, Lys-AAC substituted to Ala-GCG underline).

**Rak-HA-** The Rak cDNA was subcloned in pBluescript and obtained from Bernardu Rudi (NYU medical center). The influenza virus hemagglutinin peptide was fused to the C-terminal end of Rak essentially as described for PYK2. The oligonucleotide primers that were used in the PCR were: 5'-GCCAGCAGGCCATGTCACTGG-3' [SEQ. ID NO 11] and 5'-CGGAATTCTTACGATGCGTAGTCAGGGACATCGTATGGGTAGACATCAGTTAACATTTTG - 3'. [SEQ. ID NO 12]. The PCR product was digested with BalI and EcoRI and was used to substitute the corresponding fragment at the C-terminal end of Rak. The Rak-HA cDNA was subcloned into pRK5 downstream of the DMV promotor and into the retroviral vector pLXSN, downstream of the Mo-MuLV long terminal repeat.

### In vitro Mutagenesis

The mutagenic oligonucleotide (GAGTCAGACATCTEC-GCAGAGATTCCC) SEQ ID NO: 26 and the trans oligonucleotide GAATTCGATATCACGCGTGGCCGCCATGGC) SEQ ID NO: 27, were used to convert the tyrosine at position 402 to phenylalanine of PYK2 using a Clontech kit. Lev et *al. Nature* 376:737-745 (1995). The mutation was validated by DNA sequencing.

### Immunoprecipitation and Immunoblot Analysis

Cells were lysed in lysis buffer containing 50 mM N- 2-hydroxyethylpiperazine-N'-2-ethanesulferic acid (HEPES pH 7.5), 150 mM NaCl, 10% glycerol, 1% Triton X-100, 1.5 mM MgCl₂, 1 mM ethyleneglycol-bis (β-aminoethyl ether) -N,N,N'N'-tetraacetic acid (EGTA), 10 µg leupeptin per ml, 10 µg aprotinin per ml, 1 mM phenylmethylsulfonyl fluoride (PMSF), 200 µM sodium orthovanadate and 100 mM sodium fluoride. Immunoprecipitations were performed using protein A-sepharose (Pharmacia) coupled to specific anti-bodies. Immunoprecipitates were washed either with HNTG' solution (20 mM HEPES buffer at pH 7.5, 150 mM NaCl, 10% glycerol, 0.1% Triton X-100, 100 mM sodium fluoride, 200µM sodium orthovanadate) or successively with H' solution (50 mM Tris-HCl pH8, 500 mM NaCl, 0.1% SDS, 0.2% Triton X-100, 100 mM NaF, 200µM sodium orthovanadate) and L ' solution (10 mM Tris-HCl pH 8, 0.1% Triton X-100, 100 mM NaF, 200 µM sodium orthovanadate).

The washed immunoprecipitates were incubated for 5 min with gel sample buffer at 100°C and analyzed by sodium dodecyl sulfate polyacrylamide gel electrophoresis (SDS-PAGE). In some experiments the gel-embedded proteins were electrophoretically transferred onto nitrocellulose. The blot was then blocked with TBS (10 mM Tris pH 7.4, 150 mM NaCl) that contained 5% low fat milk and 1% ovalbumin. Antisera or purified mAbs were then added in the same solution and incubation was carried out for 1 h at 22°C. For detection the filters were washed three times (5 min each wash) with TBS/0.05% Tween-20 and reacted for 45 min at room temperature with horseradish peroxidase-conjugated protein A. The enzyme was removed by washing as described above, and the filters were reacted for 1 min with a chemiluminescence reagent (ECL, Amersham) and exposed to an autoradiography film for 1-15 min.

### In vitro kinase assay

This was carried out on immunoprecipitates in 50 µl HNTG (20 mM Hepes pH 7.5, 150 mM NaCl, 20% glycerol, 0.1% Triton X-100) containing 10 mM MnCl₂, and 5 µCi or [mN-³²P]ATP for 20 min at 22°C. The samples were washed with H',M' and L' washing solutions, boiled for 5 min in sample buffer and separated by SDS-PAGE.

### Isolation of ACK/PYK

ACK/PYK may be isolated as described in Manser et al., Nature, 363:364-367, 1993. Comparison analysis of the full length sequence of ACK/PYK with other tyrosine kinases indicates that is not closely related to any of these, although it has some similarity to the focal adhesion kinase. Therefore ACK/PYK represents a separate class of tyrosine kinases and isolation of related genes that belong to the same class is a major accomplishment.

### Example 1: Isolation of PYK-2 cDNA

To identify genes related to the ACK/PYK protein tyrosine kinase, the polymerase chain reaction (PCR) was applied in combination with degenerated oligonucleotide primers based upon conserved motifs of the kinase domain of PTKs.

Oligonucleotides primers specifically designed to a highly conserved N-terminal motif of PTKs within subdomain TK6 (IHRDLAARN) SEQ ID NO 13. and ACK/PYK specific C-terminal primers within subdomain TK9 (WMFGVTLW) SEQ ID NO 14 were utilized. The amplification reactions with cDNA templates from 8 different sources gave rise to fragments of 0.2-0.9kb. The PCR products were subcloned into pBlueScript and screened by DNA sequencing and hybridization under low stringency conditions.

A cDNA fragment of 210bp from rat spinal cord was identified which is highly related to the Focal Adhesion Kinase (FAK). The fragment was sequenced in the 3' and 5 ' directions and was subsequently used as a probe to screen cDNA libraries (human fetal brain λgt 10 and human brain λgt 11, 6x10⁵ recombinant clones each).

Several overlapping clones spreading 1.5-3kb were isolated and their cDNA inserts were analyzed by PCR, restriction mapping and sequencing. Two clones (#1 and #11) were chosen for further analysis and subcloning. Clone #1 contains an insert of 2.7kb from the 5' end of the gene, and clone #11 contains an insert of 3kb from the 3' end of the gene.

By utilizing a series of subclones and synthesized oligonucleotide primers the full length sequence of PYK-2 was determined. The sequence analysis resulted in a composite sequence of 3309bp long which contains a 104 bp 5' untranslated region, a 3021 bp coding region and 184 bp 3' untranslated region. The ATG encoding the translation initiation codon is preceded by four translation stop codons in all reading frames.

The long open reading frame encodes a protein of 1007 amino acids (predicted molecular mass of 110,770d) whose structural organization is very similar to FAK. The PYK-2 protein contains a long N-terminal sequence of 422 amino acids followed by a tyrosine kinase catalytic domain. The PYK-2 protein also contains the structural motifs common to all PTKs, two proline rich domains (19.6% and 17% proline respectively) and a focal adhesion targeting (FAT) motif in the C-terminal end. Comparison analysis of the amino acid sequence of PYK-2 with the human FAK revealed 52% identity between the two proteins. The kinase domain and the FAT sequence are most closely related (62% homology).

The PYK-2 protein contains several predicted binding sites for intracellular substrates. For example, YLMV [SEQ. ID NO 15] is a predicted binding site for GRB2 SH2 domain tyrosine 879 of PYK-2. YVVV [SEQ. ID NO 16] is a predicted binding site for SHPTP2 - tyrosine 903 of PYK-2. There are predicted phosphorylation sites for PKC, PKA and Ca/Calmodulin kinase. In addition, tyrosine 402 is a predicted autophosphorylation site of PYK-2 and it may be involved in the binding of a src SH2 domain. This is based on the homology between tyrosine 397 of FAK which was mapped as a major autophosphorylation site both *in vivo and in vitro*. This tyrosine provides a high affinity binding site for a src SH2 domain. Both tyrosine 397 of FAK and tyrosine 402 of PYK-2 are located at the juncture of the N-terminal and the catalytic domain and are followed by sequence (Y)AEI which is very similar to the consensus of the high affinity src SH2 domain binding peptide YEEI.

Total RNA from rat spinal cord was used to prepare cDNA utilizing the reverse transcriptase of Molony murine leukemia virus ('Superscript', BRL) according to the manufacturer's protocol. The cDNA was amplified by PCR utilizing degenerate oligonucleotide primers corresponding to conserved tyrosine kinase motifs from subdomains TK6 and TK9 of PYK1; (the sense and antisense primers correspond to amino acid sequences IHRDLAARN [SEQ. ID NO 171] and WMFGVTLW [SEQ. ID NO 18] respectively). The PCR was carried out under the following conditions; 1 min at 94°C; 1 min at 50°C and 1 min at 68°C for 35 cycles. Amplified DNA was subcloned and sequence, resulting in identification of a novel tyrosine kinase termed PYK2. A λgtlO human fetal brain cDNA library (clontech) was screened with ³²P-labelled PCR clone corresponding to rat PYK2. Four overlapping clones were isolated, their DNA sequence was determined on both strands utilizing series of oligonucleotide primers. The 314-bp consensus sequence contains a single open reading frame of 3027 nucleotides preceded by a 105 nucleotide 5'-untranslated region. Amino acid sequence comparisons were performed using, the Smith-Waterman algorithm of MPSRCH (IntelliGenetic) on MasPar computer.

The deduced amino acid sequence of human PYK2 from CDNA clones is shown in Figure 4. The tyrosine kinase Domain is highlighted by a dark shaded box. Two proline-rich domains in the C-terminal region are boxed with light shading. Amino acid residues are numbered on the left. Comparison of the amino acid sequence of the catalytic domain of PYK2 with four human protein tyrosine kinases demonstrated 61%, 43%, 40% and 41% sequence identity between PYK2 and Fak, Fer, HER4 and Abl, respectively. The homology between PYK2 and Fak extends beyond the catalytic domain with 42% and 36% amino acid identify in the N-terminal and C-terminal regions, respectively.

### Example 2: Pattern of PYK-2 Expression

PYK2 is highly expressed in the nervous system. We examined the expression pattern and the tissue distribution of PYK2 by Northern blot and by in *situ* hybridization analyses.

The tissue distribution of PYK-2 expression was determined by Northern blot analysis. Poly(A) RNAs were purified from mouse tissues (liver, lung, spleen, kidney, heart, brain, skin, uterus) and hybridized with two different probes corresponding to two different regions of the PYK-2 gene. The results were identical in both cases. A 4.2-4.5kb PYK-2 transcript is relatively abundant in the brain but was also found in lower levels in the spleen and in the kidney.

Film autoradiography of a sagittal section through the adult rat brain shows very high levels of expression in the olfactory bulf (OB), hippocampus (Hi), and dentate gyrus (DG). Moderate levels of expression are seen in the cerebral cortex (Cx), striatum (S), and thalamus (T) . Low levels of expression are seen in the cerebellum (Cb) and brainstem (BS).

Expression of PYK2 mRNA was determined by Northern blot analysis of poly(A)+ from various human tissues. The northern blot was hybridized with 3.9kb ³²P-labelled fragment containing the PYK2 cDNA in 50% foramide at 42°C. Northern blot of mRNA isolated from various human brain sections (amygdala, caudate nucleus, corpus callosum, hippocampus, hypothalamus, substantia nigra, subthalamic nucleus and thalamus) revealed highest expression in the hippocampus and amygdala, moderate level of expression in the hypothalamus, thalamus and caudate nucleus and low level of expression in the corpus callosum and subthalamic nucleus.

These results are consistent with in situ hybridization analysis on day 7 post natal rat brain sections utilizing antisense probes derived from PYK2 10 sequence. The *in situ* hybridization analysis demonstrate that the olfactory bulb, the hippocampus and the dentate gyrus exhibit high level of PYK2 transcripts. Moderate levels of PYK2 expression was detected in the striatum, cerebral cortex and thalamus and low levels of expression was detected in the cerebellum and brainstem.

In order to characterize the PYK2 protein, NIH3T3 cells were' transfected with a mammalian expression vector that encodes PYK2 protein with an influenza virus hemaglutanin peptide tag. PYK2 was immunoprecipicated with either anti-PYK2 or anti-HA antibodies from 3T3 transfected cell, whereas the endogenous PYK2 protein was immunoprecipitated with anti-PYK2 antibodies from PC12 cells. These antibodies precipitated a protein that migrated in SDS gels with apparent molecular weight of 112 25 kDa. Addition of Y-[32p]ATP to immunoprecipitates from PYK2 transfected cells followed by SDS-PAGE analysis and autoradiography showed that PYK2 undergoes phosphorylation on tyrosine residues.

The expression of PYK-2 in different cell lines was analyzed by IP/IB utilizing anti-PYK-2 antibodies directed to the kinase domain as described previously (GST-PYK-2). The expression pattern is summarized in table 1. Some of the interesting observations are a mobility shift of PYK-2 after differentiation of CHRF and L8057 (premegakaryocyte 35 cell lines) by TPA, high expression of Fak and PYK-2 in different cell lines; and in XC cells (rat sarcoma) PyK-2 is phosphorylated on tyrosine.

PYK2 was immunoprecipitated from NIH3T3 cells, NIH3T3 cells that overexpress PYK2-HA and PC12 cells. The immunocomplexes were washed and resolved by 7.5% SDS-PAGE. Immunoblotting was performed with anti-PYK2 antibodies. In vitro Kinase activity of PYK2. Cos cells were transiently transfected with PYK2-HA expression vector(+) or with an empty vector (-). The PYK2 protein was immunoprecipitated with anti-HA antibodies, the immunocomplexes were washed and subjected to *in vitro* kinase assay.

In situ hybridization was performed as follows: Fresh frozen rat brains were cut on a cryostat into 20-mm thick sections and thaw-mounted onto gelatin coated slides. The sections were fixed in 4% paraformaldehyde in 0.1 M sodium phosphate (pH=7.4) for 30 minutes and rinsed three times for 5 minutes each in PBS and one time for 10 minutes in 2 X SSC. Two probes were used in the hybridization analysis, a 51 base oliconucleotide complementary to the sequence encoding amino acid 301-317, and a 51 base oligonucleotide complementary to the sequence encoding amino acid 559-575 (from rat PCR product).

The oligonucleotides were labeled with a-³⁵S dATP (Du Pont-New England Nuclear) using terminal deoxynucleotidyl-transferase (Boehinger Mannheim) and purified using sephadex G-25 quick spin columns (Boehinger Mannheim). The specific activity of the labeled probes was between 5 x 10⁸ and 1 x10⁹ cpm/mg. Prehybridization and hybridization were carried out in a buffer containing 50% deionized formamide, 4 X SSC, 1 X Denhardts' solution, 500 ug/ml denatured salmon sperm DNA, 250 ug/ml yeast tRNA, and 10% dextran sulfate. The tissue was incubated for 12 hours at 45°C in hybridization solution containing the labeled probe (1 X 10⁶ cpm/section) and 10 mM dithlothreitol.

Controls for specificity were performed on adjacent sections by competitively inhibiting hybridization of the labeled oligonucleotides with a 30-fold concentration of unlabeled oligonucleotide and by hybridization with sense probes. After hybridization, the sections were washed in two changes of 2 X SSC at room temperature for 1 hour, 1 X SCC at 55°C for 30 minutes, 0.5 X SSC at 55°C for 30 minutes, and 0.5 X SSC at room temperature for 15 minutes and then dehydrated in 60, 80, 95, and 100% ethanol. After air drying, the sections were exposed to x-ray film for 5 days. The sections were then dipped in Ilford K.5 photographic emulsion (Polysciences), exposed for 4 weeks at 4°C, and developed using Kodak D- 19 developer and rapid fixer.

Emulsion autoradiography was examined by dark-field microscopy on a Zeiss axioskop. The influenza virus hemagglutinin peptide (YPYDVPDYAS) [SEQ. ID NO 19] tag was added to the C-terminal end of PYK2 utilizing the following oligonucleotide primers in the PCR: '5-CACAATGTCTTCAAACGCCAC'3' [SEQ. ID NO 20] and ' 5 - GGCTCTAGATCACGATGCGTAGTCAGGGACATCGTATGGGTACTCTGCAGGTGGGT-GGCCAG-'3' [SEQ. ID NO 21]. The amplified fragment was digested with RsrII and XbaI and used to substitute the corresponding fragment of PYK2. The nucleotide sequence of this construct was confirmed by DNA sequencing. In vitro kinase assay was carried out on immunoprecipitates in 50 µl HNTG (20 mM Hepes pH 7.5, 150 mM NaCl, 20% glycerol, 0.1% Triton X-100) containing 10 mM MnCl₂, and 5 mCi of [λ-³²P] ATP for 20 min at 22°C. The samples were washed with H' (50 mim Tris-HCI pH 8, 500 mM NaCl, 0.1% SDS, 0.2% Triton X-100, 5 mM EGTA, 100 mM NaF, 200 µM sodium orthovanadate), M' (50 mM Tris-HCl pH 8, 150 mM NaCl, 7.5 mM EDTA, 0.1% SDS, 0.2% Triton X-100, 100 mM NaF, 200 µM sodium orthovanadate) and L (10 mM Tris-HCl pH 8, 0.1% Triton X-100, 100 mM NaF, 200 µM sodium orthovanadate) washing, solutions, boiled for 5 min in sample buffer and separated by SDS-PAGE.

Antibodies against PYK2 were raised in rabbits immunized with GST fusion protein containing residues 62-647 Of PYK-2. Antibodies against influenza virus (hemagglutinin peptide) were purchased from Boehringer Mannheim. Cell lysis, immunoprecipitations and immunoblotting was performed essentially as described by Lev et al. Mol. Cell. Biol. 13, 2224-2234, 1993.

### Example 3: Properties of PYK-2 protein

In order to analyze the biochemical properties of PYK-2 the full length cDNA was subcloned into the two mammalian expression vectors RK5 and pLSV. In parallel, an expression vector encoding the PYK-2 protein fused to the influenza virus hemagglutinin peptide was constructed. This construct was used to identify the protein utilizing anti-HA antibodies.

pLSV-PYK-2-HA was transfected into. cos cells. The protein was expressed at the predicted molecular mass(-116kD) as determined by IP and IB with anti-HA antibodies. The protein is an active kinase as determined by in vitro kinase assay utilizing (^{λ32P}) ATP or an in vitro kinase assay utilizing cold ATP and immunoblotting with anti-phosphotyrosine antibodies.

The PYK-2 cDNA cloned in pLSV was cotransfected with pSV2neo into PC12 cells and NIH3T3 in order to establish stable cell lines. G418 resistant colonies were screened by immunoprecipitating and immunoblotting.

NIH3T3 cell lines were established that overexpress the PYK-2 and the PYK-2-HA protein. In these cells PYK-2 undergoes tyrosine phosphorylation in response to PDGF, EGF and aFGF. The level of phosphorylation is not so high. The stronger effect is achieved by TPA treatment (6µM) after 15 min incubation at 37°C as determined by time course analysis.

### Example 4: Tyrosine phosphorylation of PYK2 in response to carbachol, membrane depolarization and Ca+2 influx.

The phosphorylation of PYK-2 on tyrosine residue in response to different stimulus was analyzed by immunoprecipitation of PYK-2 and immunoblotting with anti-phosphotyrosine antibodies and vice versa.

The following treatments were used: Bradykinin, TPA, forskolin, forskolin + TPA, bradykinin + forskolin, NGF, Neuropeptide Y, Cholera toxin, Cholera toxin+TPA, Cholera toxin + bradykinin, pertusis toxin, pertusis toxin + TPA, bradykinin + pertusis toxin, calcium ionophore A23187, bombesin.

The following results were obtained: PYK-2 undergoes tyrosine phosphorylation in response to TPA (1.6µM 15 min at 37°C), bradykinin (1µM 1 min at 37°C) and calcium ionophore A23187 (2µM 15 min at 37 °C). Forskolin increases the response of TPA but does not give any signal by itself. Cholera toxin gave higher signal in combination with TPA and bradykinin but didn't cause phosphorylation of PYK-2 alone. Pertusis toxin also induced the response of TPA and bradykinin but didn't cause any response alone. In order to determine if the bradykinin effect is mediated by PKC signaling pathway attempts to down regulate PKC by chronic treatment with TPA (twice) did not give a clear answer. One interpretation of these results is that PKC and PKA (and maybe Ca/calmudolin kinase) induce the autophosphorylation of PYK-2 in response to ser/the phosphorylation. This interpretation may be checked by utilizing specific inhibitors to PKC and PKA and by phosphamino-acid analysis.

Confluent PC12 cells in 150mm plates were grown for 18 hours in DMEM containing 0.5% horse serum and 0.25% fetal bovine serum. The cells were sitmulated at 37°C with different agonists as indicated, washed with cold PBS and lysed in 800ml lysis buffer (Lev et al., supra). The cell lysates were subjected to immunoprecipitation with anti-PYK2 antibodies. Following SDS-PAGE and transfer to nitrocellulose the samples were immunoblotted with either antiphosphotyrosine (RC20, transduction laboratories) or anti-PYK2 antibodies.

Carbachol induces tyrosine phosphorylation of PYK2 via activation of the nicotinic acetylcholine receptor. Immunoprecipitates of PYK2 from PC12 cells that were subjected to the following treatments: muscarine (ImM) or carbachol (ImM) for 20 sec at 37°C. Carbachol (1mM), DMPP (100µM), or carbachol after pretreatment with the muscarinic antagonist atropine (100nM) or the nicotinic antagonist mecamylamine (10µM) for 5 min at 37°C. Incubation with carbachol in the presence or absence of EGTA (3mM) as indicated. The immunocomplexes were resolved by SDS-PAGE, transferred to nitrocellulose, and probed with either anti-phosphotyrosine antibodies or with anti-PYK2 antibodies as indicated. Membrane depolarization and calcium ionophore induce tyrosine phosphorylation of PYK2. Immunoprecipitates of PYK2 from quiescent PC12 cells were subjected to the following treatments: incubation with 75mM KCI in the presence or absence of EGTA (3mM), incubation with 6µM of the calcium ionophore A23187 for 15 min at 37°C. The immunoprecipitates were washed, resolved by 7.5% SDS-PAGE and immunoblotted with either antiphosphotyrosine antibodies or with anti-PYK2 antibodies.

Activation of PYK2 by carbachol, membrane depolarization and Ca+2 influx was studied. Since PYK2 is highly expressed in the central nervous system and in PC12 cells, we examined the effect of a variety of neuronal agonists on the phosphorylation state of PYK2. In these experiments, PC12 cells were treated with an agonist, lysed and subjected to immunoprecipitation with anti-PYK2 antibodies followed by SDS-PAGE analysis and immunoblotting with phosphotyrosine specific antibodies.

Stimulation of PC12 cells with carbachol induces strong, tyrosine phosphorylation of PYK2. We explored the possibility whether activation of both cholinergic receptor subtypes leads nicotinic and muscarinic receptors to tyrosine phosphorylation of PYK2. Pharmacological analysis with either subtype specific agonists, muscarine and DMPP or subtype specific antagonists, atropine and mecamylamine indicated that activation of PYK2 by carbachol is mediated via the nicotinic acetylcholine receptor. The phosphorylation of PYK2 in response to carbachol is very rapid; 5 seconds after applying carbachol to the cells, PYK2 became phosphorylated on tyrosine residues. Elimination of extracellular calcium by EGTA completely blocked agonist induced tyrosine phosphorylation of PYK2, indicating that calcium influx is required for carbachol induced PYK2 activation.

Stimulation of the nicotinic acetylcholine receptor induces membrane depolarization by cation influx via the ion channel pore. We have therefore checked whether membrane depolarization induced by a high concentration of potassium chloride will cause the same effect on PYK2 tyrosine phosphorylation. Depolarization of PC12 cells with 75mM KCl induces rapid tyrosine phosphorylation of PYK2. The omission of calcium from the extracelluar medium completely abolished PYK2 tyrosine phosphorylation, indicating that activation of PYK2 is due to calcium influx rather than membrane depolarization per se. To further explore this possibility, we examined the effect of a calcium ionophore on PYK2 activation. PYK2 is phosphorylated on tyrosine residues following incubation with the calcium ionophore A23187. These results show that elevation of intracellular calcium in response to a variety of stimuli causes tyrosine phosphorylation of PYK2.

Tyrosine phosphoyrlation of PYK2 in response to activation of a G protein coupled receptor was studied. We analyzed the effect of bradykinin on the phosphorylation state of PYK2. Bradykinin induces rapid tyrosine phosphorylation of PYK2 in PC12 cells. By contrast to stimulation of PYK2 phosphorylation in response to carbachol treatment or to membrane depolarization the effect of bradykinin was not influenced by the omission of extracellular calcium; bradykinin induced PYK2 phosphorylation in the absence of extracellular calcium or in the presence of EGTA.

Incubation of PC12 cells with phorbol myristate acetate (PMA) induced tyrosine phosphorylation of PYK2, suggesting that tyrosine phosphorylation of PYK2 could also be mediated via protein kinase C (PKC) activation. To determine whether bradykinin-induced phosphorylation of PYK2 is mediated via PKC, the cells were treated with bradykinin or PMA following down-regulation of PMA-sensitive PKC isozymes by prolonged treatment with PMA. Prolonged treatment with PMA completely abolished the effect of PMA, but had only a minor effect on bradykinin-stimulated tyrosine phosphorylation of PYK2. These results suggest that tyrosine phosphorylation of PYK2 can be induced by PKC-independent and by PKC-dependents mechanisms.

### Example 5: Phosphorylation of RAK

293 cells in 65mm plates were transiently transfected either with the potassium channel-RAK-HA alone, or together with Fak, PYK2 or the PYK2-kinase negative mutant (PKN). 12 hr following transfection the cells were grown in DMEM containing 0.3% fetal bovine serum for 24 hours. The cells were either stimulated with PMA (1.6µM) or with calcium ionophore A23187 (6µM) for 15 min at. 37°C or left unstimulated. The cells were solubilized and the expression level of each protein was determined by western blot analysis. The Rak protein was immunoprecipitated by anti-HA antibodies and its phosphorylation on tyrosine residues was analyzed by western blot analysis utilizing anti-phosphotyrosine antibodies following immunoprecipitation of the proteins either with anti-PYK2 antibodies (for PYK2 and PKN) or with anti Fax antibodies for (Fak).

The expression level of each protein (Rak PYK2, PKN and Fak) and the tyrosine phosphorylation of Rak, PYK2, PKN and Fak were measured. Only the kinase active PYK2 protein phosphorylated the potassium channel. No phosphorylation was observed with kinase negative PYK2 or with FAK.

### Example 6: Tyrosine Phosphorylation of PYK2 and Shc in response to activation of PC 12 cells by different stimuli.

PC12 cells were grown in DMEM containing 0.25% fetal bovine serum and 0.5% horse serum for 18 hours before stimulation. Following stimulation, the cells were washed with cold PBS and lysed in 0.8 ml lysis buffer (Lev et al., Mol. Cell. Biol. 13, 225-2234, 1993). PYK2 was immunoprecipitated by anti-PYK2 antibodies, the immunoprecipitates were resolved by 7.5% SDS-PAGE and immunoblotted either with anti-phosphotyrosine antibodies (RC20, transduction laboratories) or with anti-PYK2 antibodies. Antibodies against PYK2 were raised in rabbits.

Tyrosine phosphorylation of PYK2 in response to different stimuli was studied. Quiescent PC12 cells were stimulated at 37°C with carbachol (ImM, 20 sec), bradykinin (lµM, lmin KCI (75mM, 3 min) , PMA (1.6µM, 15min), A23187 (6µM, 15min) or left unstimulated (-) . PYK2 was immunoprecipitated from cell lysates with anti-PYK2 antibodies, followed by SDS-PAGE and immunoblotting, with anti-phosphotyrosine or anti-PYK2 antibodies. Tyrosine phosphorylation of Shc in response to bradykinin, carbachol, PMA and other stimuli was also measured. Quiescent PC12 cells were stimulated for 5 min at 37°C with bradykinin (1µM),carbachol (ImM) , KCl (75mM), PMA (1.6µM), NGF(100ng/ml), or left unstimulated (-). The cells were also stimulated with carbachol (ImM) or potassium chloride (75mM) in the presence of 3mM EGTA. Stimulations with DMPP (100µM) or muscarine (ImM) were performed under the same conditions. Time-course of carbachol induced tyrosine phosphorylation *of* Shc was performed by incubation of the cells with 1mM carbachol. The Shc proteins were immunoprecipitated with anti-Shc antibodies, the immunoprecipitates were resolved by SDS- PAGE(8%), transferred to nitrocellulose and immunoblotted with anti-phosphotyrosine antibodies.

### Example 7: Association of PYK2 with Grb2 and Sos 1 in intact cells.

In order to explore the possibility that calcium induced PYK2 activation is responsible for tyrosine phosphorylation of Shc and activation of the Ras/MAPK signaling pathway, we have examined the ability of PYK2 to recruit upstream regulatory elements of this signaling, pathway, such as Shc and Grb2. Human embryonic 293 cells were transiently transfected with different combinations of expression vectors that direct the synthesis of PYK2,a kinase negative PYK2 mutant (PKN) and the adaptor protein Grb2. The results show that Grb2 is directly associated with wild type PYK2 but not with the kinase negative mutant. Experiments with GST-fusion protein of Grb2 indicate that the association between Grb2 and PYK2 is mediated via its SH2 domain. Inspection of PYK2 primary structure shows that tyr881 is followed by a LNV sequence which was shown to be a canonical binding site for the SH2 domain of Grb219.

We next examined the interaction of PYK2 with the gauanine nucleotide releasing factor SOS I. Human embryonic kidney 293 cells were transfected with expression vectors encoding SOS 1, PYK2 and PKIN and subjected to immunoprecipitation/immunoblotting analysis with anti Sosl or anti-PYK2, antibodies, respectively. Wild type PYK2 but not the kinase negative mutant (PKN) was co-immunoprecipitated with the with Sosl protein. Hence, Grb2 is bound to Sosl via its SH3 domains and to PYK2 via its SH2 domain leading to the recruitment of Sos by tyrosine phosphorylated PYK2.

Growth factor induced activation of receptor tyrosine kinases leads to a shift in the electrophoretic mobility of SOS protein. The mobility shift was shown to be due to phosphorylation by serine and thronine kinases which are dependent upon Ras activation including the MAP kinase 11, 12, 20. SOS I protein from PYKI- transfected cells exhibits reduced electrophoretic mobility as compared to SOS I protein. This experiment shows that PYK2 over-expression leads to the activation of the ser/thr kinases responsible for the phosphorylation of SOS 1.

293 cells were transiently transfected with the full length cDNAs of PYK2, PKN Grb2 and hSosl-HA cloned into the mammalian expression vectors pRK5 downstream to the CMV promotor, using the calcium phosphate precipitation method (Wigler et al., Cell 16, 777-785, 1979). Twelve hours after transfection, the cells were incubated in medium containing 0.2% fetal bovine serum for 24 hours. The cells were lysed, subjected to immunoprecipitation, resolved by SDS-PAGE (15% for Grb2 IPS, 7.5% for PYK2 IPS) and immunoblotted essentially as described (Lev et al., Mol. Cell Biol. 13, 2224-2234, 1993). For immunoblotting we used a mouse monoclonal antibody against Grb2 (Transduction laboratories #GI6720). The kinase negative mutant of PYK2 was constructed as described. A mammalian expression vector encoding the hSosl-HA was constructed as described (Aronheim et al., Cell 78, 949-961, 1994).

Embryonic human kidney 293 cells were transiently transfected with different combinations of mammalian expression vectors that direct the synthesis of Grb2, PYK2 and a kinase negative PYK2 point mutant (PKN). The cells were solubilized and immunoprecipitated with anti-Grb2 or anti-PYK2 antibodies. The immunocomplexes were washed, resolved by SDS-PAGE, transferred to nitrocellulose and immunoblotted with either anti-PYK2 or anti-Grb2 anti-bodies. The expression level of Grb2 in each cell line was determined by immunoblotting of total cell lysates with anti-Grb2 antibodies.

Embryonic human kidney 293 cells were transiently transfected with mammalian expression vectors encoding hsosl-HA, hSosl-HA together with PYK2 or hSosl-HA together with PKN. Hsosl was immunoprecipitated with anti HA antibodies from each cell line, and the presence of PYK2 in the immunocomplexes was determined by immunobloting with anti-PYK2 antibodies. Expression levels of hsosl, PYK2 and PKN were determined by immunoblot analysis of total cell lysates, with anti-HA or anti PYK2 antibodies.

### Example 8: PYK2 induces tyrosine phosphorylation of Shc and its association with Grb2.

Activated EGF receptor is able to recruit Grb2 directly and indirectly. We have therefore investigated whether PYK2 can induce phosphorylation of Shc tyrosine phosphorylation of Shc and its association with Grb2. Shc proteins were immunoprecipitated with anti Shc antibodies from Shc, from Shc and PYK2, or from Shc and PKIN expressing cells. The samples were resolved by SDS-PAGE, transferred to nitrocellulose and immunoblotted with anti-phosphotyrosine or anti-Grb2 antibodies. Dramatic tyrosine phosphorylation of Shc in cells that overexpress PYK2. Moreover, several phosphotyrosine containing proteins were found in Shc immunoprecipitates from PYK2 overexpressing cells. Similar results were observed in cells expressing endogenous Shc proteins that were transfected with PYK2 CDNA and subject to immunoprecipitation analysis with anti Shc antibodies. Immunoblot analysis with Grb2 antibodies of Shc immunoprecipitates indicted that Grb2 associates with tyrosine phosphorylated Shc in PYK2 overexpressing cells. We therefore conclude that tyrosine phosphorylated PYK2 can directly and indirectly recruit Grb2 via tyrosine phosphorylation of Shc revealing at least two alternative routes for pYK2 induced activation of the Ras signaling pathway.

Tyrosine phosphorylation of Shc in cells that coexpress YK2 was standard. Cells that express Shc alone or coexpress Shc together with either PYK2 or PKN were lysed and subjected to immunoprecipitation with anti-Shc antibodies or pre-immune serum (P.I.). The immune-complexes were washed, run on an SDS gel and immunoblotted with anti-phosphotyrosine antibodies. Shc proteins (46,52 and 66kDa) were identified.

PYK2 induces association of Shc with Grb2. Shc proteins were immunoprecipitated from each cell line using anti-Shc antibodies. As a control, the lysates of cells that coexpress PYK2 and Shc were subject to immuno.-precipitation with pre-immune serum (P.I.). The presence of Grb2 in the immunocomplexes was determined by immuno-blotting with anti-Grb2 antibodies.

The expression level of of PYK2, PKN and Shc in each cell line was determined by immunoblot analysis of total cell lysates with specific antibodies as indicated.

### Example 9: Activation of MAP kinase in PC12 cells by bradykinin, carbachol and other stiumli.

The experiments presented so far show that the same stimuli that induce activation of PYK2 induce tyrosine phosphorylation of Shc. We next examined the ability of these agents to induce the activation of kinases in PC12 cells. Quiescent PC12 cells were incubated with a variety of stimuli. Lysates from stimulated 'cells were subjected to immunoprecipitation with anti-MAP kinase antibodies followed by immunoblotting, with phosphotyrosine antibodies. Myelin basic protein (MBP) was utilized as a substrate to determine MAP kinase activation. The addition of various ligands to PC12 cells induced a similar profile of both tyrosine phosphorylation and activation of MAP kinase in these cells.

Since activation of MAP kinase was observed in response to stimuli that induce PYK2 phosphorylation, we examined the possibility whether PYK2 overexpression can induce MAP kinase activation. Human embryonic kidney 293 cells were transiently transfected with increasing concentrations of mammalian expression vector that directs the synthesis of PYK2. The cells were grown for 24 hours in the presence of 0.2% serum, MAPK 1,2 proteins were immunoprecipitated, washed and subjected to MBP phosphorylation assay. The results presented in figure 4b show that PYK2 overexpression induced MBP phosphorylation in a concentration dependent manner.

Quantitation of these results shows that MAP kinase activity was approximately three fold higher in cells that expressed the highest level of PYK2 as compared to mock transsfected cells.

293 cells were transiently transfected with mammalian expression vectors for Shc alone, Shc together with PYK2, or Shc together with PKN. PC12 cells were starved for 18 hours as described. The cells were stimulated for 5 min at 37°C with the indicated stimuli, lysed and subjected to immunoprecipitation with antiMAPK 1,2 antibodies (Santa Cruz Biotechnoloay, #c-14 and #c-16). The immunoprecipitates were washed twice with lysis buffer (Lev et al., Mol. Cell. Biol. 13, 2224-2234, 1993) and once with Tris-buffer containing 10mM Tris-HCI pH 7.2, 100mM NaCl, 1mM Na-vanadate and 5mM benzamidine. The immunocomplexes were resuspended in 40µl of MAP kinase-buffer containing 30mM Tris-HCI pH 8, 20mM MgCl₂, 2mM MnCl₂, 15µg, MBP, 10µM ATP and 5µCiτ-[³²P]ATP (Amersham).The samples were incubated for 30 min at 30°C and the reactions were stopped by the addition of SDS-sample buffer. The samples were resolved on 15% SDS-PAGE and analysed by autoradiography. Human embryonic kidney 293 cells were transiently transfected with increasing concentration of pRK5-PYK2 DNA (0.5-µg). Twelve hours after transfection the cells were grown in medium containing 0.2% serum for 24 hours. The cells were lysed, immunoprecipitated with MAPK 1,2 antibodies and subjected to MBP phosphorylation assay as described above.

Quiescent PC12 cells were stimulated for 5 min at 37°C with bradykinin (1µM), carbachol (ImM), KCl (75mM), PMA (1.6µM), NGF (100mg/ml), or left unstimulated (-) . The cells were lysed and MAPK 1,2 were immunoprecipitated with specific antibodies. The immunocomplexes were washed and ither resolved by SDS-PAGE, transferred to nitrocellulose and immunoblotted with anti-phosphotyrosine antibodies, or subjected to a standard myelin basic protein (MBP) phosphorylation assay.

Activation of MAP kinase by overexpression of PYK2. Human embryonic kidney 293 cells were transiently transfected with increasing concentrations of a mammalian expression vector that directs the synthesis of PYK2. MAPK 1,2 proteins were immunoprecipitated from each cell line, the immunocomplexes were washed and subjected to MBP phosphorylation assay. Quantitation of MAP kinase activity for each cell line was determined by phosphorimager and ImagQuant software (Molecular Dynamics, Incorporated). MAPK activity in transfected cells is compared to activity detected in control mock transfected cells.

### Example 10: Bradykinin stimulation of PC12 cells induces tvrosine phosphorvlation of PYK2.

Ligand stimulation, immunoprecipitations and immuno-blotting were performed. Chronic treatment with PMA was performed by incubation of the cells with 100nM PMA for 12 hours at 37°C.

Time course of bradykinin induces tyrosine phosphorylation of PYK2. Quiescent PC12 cells were incubated at 37°C with 1µM bradykinin for indicated periods of time. PYK2 was immunoprecipitated from untreated (-) or treated cells, the immunocomplexes were washed, resolved by SDS-PAGE, transferred to nitrocellulose, and probed either with anti-phosphotyrosine or anti-PYK2 antibodies.

Quiescent PC12 cells were incubated with either 1µM bradykinin (1 min at 37°C) or with PMA (1.6µM, 15 min at 37°C) in the presence or absence of CaCl or EGTA (3µM) as indicated. In some cases the cells were pretreated with 100nM PMA for 12 h. PYK2 was immunoprecipitated from stimulated or unstimulated cells (-) and analysed by immunoblot analysis with either anti-phosphotyrosine or anti-PYK2 antibodies.

### Example 11: Stimulation of Kv1.2 potassium channel tvrosine DhosDhorvlation in response to PYK2 activation.

We examined the possibility whether PYK2 can tyrosine phosphorylate the Kv1.2 channel and regulate its function. In order to test this possibility we expressed in 293 cells the Kv1.2 protein, Kv1.2 together with PYK2, and as a control Kv1.2 with a kinase negative PYK2 mutant (PKN) or with the protein tyrosine kinase Fak. The cells were grown for 24 hours in medium containing 0.2% serum and then stimulated with PMA(1.6µM), calcium ionophore (6µM), or left unstimulated.

Immunoblotting analysis with phosphotyrosine anti-bodies following immunoprecipitation of PYK2, PKN and Fak by specific antibodies. PYK2 and Fak were phosphorylated on tyrosine even in unstimulated cells and treatment with PMA induced tyrosine phosphorylation while treatment with calcium ionophore induced a weaker response. The level of expression of the kinase negative mutant of PYK2 (PKN) was similar to the expression of wild type PYK2 or FAK.' Nevertheless, as expected, PKN was not found to be phosphorylated on tyrosine residues. We next analyzed the tyrosine phosphorylation of Kv1.2 channel in each cell line.

We have added to the cDNA expression construct of Kv1.2 a HA tag and determined the level of Kv1.2 expression by immunoblot analysis with anti-HA antibodies. A similar amount of Kv1.2 protein was expressed in the transfected cell lines. The Kv1.2 protein was immune-precipitated from unstimulated cells as well as from PMA or calcium ionophore stimulated cells. The immunoprecipitates were resolved by SDS-PAGE and immunobloted with anti-phosphotyrosine antibodies. Phosphorylation of Kv1.2 on tyrosine residues was observed only in cells coexpressing PYK2. Moreover, tyrosine phosphorylation of Kvl.2 was enhanced by PMA or calcium ionophore treatments indicating that activation of PYK2 is required for PYK2 in duced tyrosine phosphorylation of the potassium channel.

Embryonic human kidney 293 cells were transiently transfected with different combinations of mammalian expression vectors which direct the synthesis of Kv1.2-HA, PYKZ, a kinase negative PYK2 (PKN) or the protein tyrosine kinase Fak. The cells were grown for 24 h in the presence of 0.2% serum and then either stimulated with PMA (1.6µM, 10 min at 37°C), the calcium ionophore A23187 (6µM, 10 min at 37°C) or left unstimulated (-). Tyrosine phosphorylation of each protein was analysed following immunoprecipitation and immunoblotting with anti-phosphotyrosine antibodies. The expression of each protein was determined by immunoblot analysis of total cell lysates from each transfection with anti-PYKZ, anti-HA or anti-Fak antibodies.

Tyrosine phosphorylation of Kv1.2 was analysed by immunoprecipitation of Kv1.2-HA protein from each cell line with anti-HA antibodies, followed by immunoblot analysis with anti-phosphotyrosine antibodies. 293 cells were transfected by the calcium phosphate technique as described (Wigler et al., Cell 16, 777-785, 2979). The influenza virus hemagglutinin peptide (YPYDVPDYAS) [SEQ. ID NO 22] tag was added to the C-terminal end of the Kv1.2 cDNA utilizing the following oligonucleotide primers in the PCR;
'5GCCAGCAGGCCATGTCACTGG-3' [SEQ.IDNO23] and '5CGGAATTCTTACGATGCGTAGTCAGGGACATCGTATGGGTAGACATCAGTTAAC-ATT TTG-'3 [SEQ. ID NO 24]. The PCR product was digested with BAIT and EcoRI and used to substitute the corresponding fragment at the C-terminal end of the Kv1.2 cDNA. The Kv1.2-HA cDNA was subcloned into pRK5 downstream of the CMV promotor.

A kinase negative mutant of PYK2 (PKN) was constructed by replacing Lys475 with an Ala residue by utilizing a site directed mutagenesis Kit (Clontech). The oligonucleotide sequence was designed to create a new NruI restriction site. The nucleotide sequence of the mutant was confirmed by DNA sequencing. The oligonucleotide sequence that was used for mutagenesis is:
'5-CAATGTAGCTGTCGCGACCTGCAAGAAAGAC-3' [SEQ. ID NO 25] (NruI site - bold, Lys-AAC substituted to Ala-GCG underline). The full length cDNAs of PYK2, PKN and Fak were subcloned into the mammalian expression vectors pRK5 downstream to the CMV promotor.

### Example 12: Suppression of potassium channel action in frog oocytes by PYK2 expression and PMA treatment.

In vitro capped RNA transcripts of Kv1.2, PYK2 and PKN were synthesized from linearized plasmids DNA templates utilizing the mMESSAGE mMACHINE kit (Ambion), following the supplier's protocols. The products of the transcription reaction (cRNAs) were diluted in RNAse-free water and stored at -70°C. Expression of the RNAs was done by injection of 50nl of RNA into defolliculated stage V and VI oocytes from Xenopus laevis (Iverson et al., J. Neurosc. 10, 2903-2916, 1990). The injected oocytes were incubated for 2-3 days at 20°C in L15 solution (1:2 dilution of Gibco's Leibovitz L15 medum in H20, with 50U/ml nystatin, O.lmg/ml gentamycin, 30mM HEPES buffer, pH 7.3-7.4, filtered through a 0.45mm membrane). Electrophysiological Recording and analysis. Ionic currents were recorded with a two microelectrode voltage-clamp as described (Iverson et al., supra). The current were low-pass filtered KHz using an 8-pole Bessel filter and stored in a 80286 microcomputer using the pClamp acquisition system (Axon Instruments). The data was analyzed with the clamp fit pro-rams of the pCIamp system (Axon Instruments). All recording were performed at room temperature (20-230°C). The recording chamber was continually perfused with recording solution. To avoid contamination of the oocyte by Ca+²-activated Cl⁻ currents low Cl⁻ recording solution was used (96mM Na+, glutamate, 2mM K⁺ glutamate, 0.5mM CaCl₂, 5mM MgCl₂, 5mM HEPES buffer). The Kt currents were elicited in depolarizinc, steps from -100 to +40 mv in 10 mV increments every 15 seconds. Kv1.2 currents from oocytes microinjected with either Kv1.2 mRNA, Kv1.2 and PYK2 mRNAs, or Kv1.2 and a kinase negative mutant of PYK2 mRNAs (PKN). Currents were elicited in response to depolarizing steps from - 100 to ⁺ 30 mV increments from a holding potential of -110 mV. Representative traces of Kv1.2 channels before and after bath application of 100 nM PMA at the annotated time (8 and 20 minutes) in the same cell.

Suppression of Kv1.2 currents in response to PMA is blocked by a kinase negative PYK2 mutant (PKN). Inhibition of Kvl.2 currents in an oocyte microinjected with Kv1.2, mRNA before and 25 minutes after treatment with PMA at 50 nM or 100 nM concentration. Recordings from an oocyte expression Kv1.2 and PYK2 or Kv1.2 and a kinase negative mutant of PYK2 under the same conditions as described above. The same protocol was utilized in both experiments.

We asked whether stimulation of PYK2 can suppress Kv1.2 currents. We explored the effect of PYK2 expression, on currents exhibited by Kv1.2 expression in Xenopus oocytes. Stage V oocytes were microinjected either with Kv1.2 transcripts or with Kv1.2 together with PYK2 or PKN mRNAs. Following two to three days of incubation at 20°C, macroscopic currents exhibited by the oocytes were recorded with a two microelectrode voltage clamp as described (Iverson et al., J. Neurosc. 10, 2903-2916, 1990). Outward rectifier currents were recorded upon membrane depolarization above -40mV, indicating that a functional Kv1.2 channel is expressed in the oocytes. The expression of Kv1.2, PYK2 and PKN in the frog oocytes was confirmed by immunoblot analysis with anti-HA or anti-PYK2 antibodies.

We have examined the effect of PYK2 expression on Kv1.2 currents in oocytes in the absence or presence of PMA. We also examined the effect of the kinase negative mutant PKN on PMA induced suppression of Kv 1.2 currents mediated by the endogenous protein tyrosine kinase. Treatment of oocytes with PMA caused inhibition of Kv1.2 currents. As previously shown, the inhibition of the currents developed gradually after application of PMA reaching 80-90% inhibition after 20 min incubation (Huang et al., Cell 75, 1145-1156, 1993). Moreover, the rate of channel blockade was found to be dependent upon the concentration of PMA applied. Coexpression of PYK2 resulted in acceleration of Kv1.2 currents inhibition. Significant acceleration of current inhibition was observed at every concentration of PMA tested. For example, 8 min after the addition of 100 nM PMA, 25% inhibition of outward current was observed in oocytes expressing Kv1.2 alone as compared to 95% inhibition observed in oocytes coexpressing Kv1.2 and PYK2 proteins.

Current inhibition by PMA treatment in the absence or presence of PYK2 expression did not result in changes in both the kinetics or voltage dependence of the remaining currents. Coexpression of Kvl.2 together with the kinase negative mutant of 'PYK2 (PKN) led to nearly complete inhibition of PMA induced potassium channel blockage. It is possible that the endogenous protein tyrosine kinase activated by PMA that is responsible for suppression of Kv1.2 currents in oocytes represents the xenopus homologue of PYK2 or a closely related protein tyrosine kinase that can be affected by a dominant interfering mutant of PYK2.

### Example 13: PYK2 is Phosphorylated upon LPA and Bradykinin Stimulation of Cells

We have now demonstrated that lysophosphatidic acid (LPA) and bradykinin do in fact induce tyrosine phosphorylation of PYK2 as well as complex formation between PYK2 and activated Src. This observation provides novel strategies to screen for modulators of PYK2 signalling pathways. Moreover, tyrosine phosphorylation of PYK2 leads to binding of the SH2 domain of Src to the tyrosine at position 402 of PYK2 and activation of Src, thereby providing even further information useful in the rational design of such PYK2 signalling pathway modulators. Transient overexpression of a dominant interfering mutant of PYK2 or the protein tyrosine kinase Csk reduces LPA- or bradykinin-induced activation of MAP kinase. LPA- or bradykinin-induced MAP kinase activation was also inhibited by overexpression of dominant interfering mutants of Grb2 and Sos. Thus, without wishing to be bound to any particular theory of the invention, we propose that PYK2 acts in concert with Src to link Gi- and Gq- coupled receptors with Grb2 and Sos to activate the MAP kinase signalling pathway in PC12 cells.

PC12 cells were stimulated with lysophosphatidic acid (LPA) and lysates of stimulated or unstimulated cells were subjected to immunoprecipitation with antibodies against PYK2 followed by immunoblotting with antibodies against phosphotyrosine. The experiment shows rapid tyrosine phosphorylation of PYK2 in response to LPA or bradykinin stimulation. Pretreatment of PC12 cells with pertussis toxin significantly inhibited tyrosine phosphorylation of PYK2 in response to LPA stimulation. However, bradykinin or phorbol 12-myristate 13-acetate (PMA) induced activation of PYK2 were not affected by pretreatment with pertussis-toxin. Removal of extracellular calcium by the addition of EGTA to the medium did not affect LPA-induced PYK2 phosphorylation. These results indicate that LPA-induced PYK2 activation is dependent, at least in part, on a pertussis-toxin sensitive Gi-dependent pathway in PC12 cells.

PC12 cells were stimulated with LPA (2.5mM) or bradykinin (1 mM) for 3 minutes at 37°C and lysed. PYK2 was immunoprecipitated with antibodies against PYK2 and immunoblotted with antibodies against phosphotyrosine (anti-pTyr) or against PYK2.

PC12 cells were left untreated (-) or incubated with pertussis toxin (PTx) 100 ng/ml for 20 hours and then stimulated with LPA (2.5 mM), bradykinin (1 mM) for 3 min at 37°C or with PMA (1 mM) for 10 min at 37°C. In addition, cells were stimulated with LPA in medium containing 3 mM EGTA. We have analyzed phosphorylation of immunoprecipitated PYK2 by immunoblotting with anti-pTyr or anti-PYK2 antibodies. LPA, PMA, PMA, and bradykinin yielded 4, 12, and 9 fold increases in the phosphorylation state of PYK2 relative to unstimulated cells.

PC12 cells were transiently transfected with pRK5, PYK2 kinase negative mutant (PKM)and Csk. Expression of Csk was determined by blotting total cell lysates with anti-Csk antibodies PC12 cells were mock transfected (pRK5), or trans fected with truncated EGF receptor, PKM, Csk, or with both PKM and Csk, stimulated with LPA or bradykinin for 3 min, lysed and MAP kinase activity was determined. The trans fection efficiency was determined using a b-Galactosidase transgene. Similar results were obtained in three independent experiments performed in duplicate. MAP kinase activity in PC12 cells over-expressing dominant interfering mutants of Grb2 and Sos was also assessed. Cells were stimulated with LPA or bradykinin and MAP kinase activity was determined. The experiments were repeated three times.

### Example 14: PYK2 and Src Associate upon LPA and Bradykinin Stimulation of Cells

We have demonstrated that activation of PYK2 by elevation of intracellular calcium concentration can lead to activation of the MAP kinase signaling pathway in PC12 cells. Src family protein tyrosine kinases are activated in response to stimulation of a variety of G protein-coupled receptors and have been shown to be necessary for linking Gi- and Gq-coupled receptors with MAP kinase activation. Sadoshima & Izumo, *EMBO J.* 15:775-787 (1996) ;Wan *et al ., Nature* 380:541-544 (1996). The EGF-receptor and erbB2 were also implicated in MAP kinase activation induced by LPA and other agonists of G-protein coupled receptors. Daub *et al .* , *Nature* 379:557-564 (1996). We have tested whether LPA and bradykinin can activate Src and the EGF receptor in PC12 cells. Stimulation of PC12 cells by LPA or bradykinin leads to approximately three to four fold increase in Src kinase activity, while we were unable to detect LPA-induced activation of EGF receptor in PC12 cells.

We examined the possibility of association between the two protein tyrosine kinases PYK2 and Src in response to LPA or bradykinin stimulation. Lysates from stimulated or unstimulated cells were subjected to immunoprecipitation with antibodies against Src followed by immunoblotting with antibodies against PYK2, against Src or anti-pY416^{src} antibodies that specifically recognize activated.*Src*. *Liu et al ., Oncogene* 8:1119-1126 (1993) . This experiment demonstrated that PYK2 forms a complex with activated Src in response to LPA or bradykinin stimulation. Furthermore, the SH2 domain of Src bound to activated PYK2 suggesting that Src binding to PYK2 is mediated by means of its SH2 domain perhaps leading to its activation.

To further analyze the interaction between Src and PYK2, we performed an *in vitro* binding experiment using a GST fusion protein containing the SH2 domain of Src with wild-type PYK2, a mutant form of PYK2 in which the tyrosine at position 402 was replaced by a phenylalanine residue (PYK2-Y402F) or a kinase negative mutant of PYK2. Lev *et al*., 1995, *Nature* 376:737-745. A GST fusion protein containing the SH2 domain of Src bound to tyrosine phosphorylated PYK2 but not to the PYK2-Y402F mutant or PKM. The tyrosine at position 402 represents the major autophosphorylation site of PYK2 and is found within the sequence YAEI,a consensus binding site for the SH2 domain of Src kinases Songyang *et al., Cell* 72:76-778 ( 1993 ).

PC12 cells were left untreated (-)or stimulated with LPA (2.5mM) or bradykinin (1mM) for 3 min at 37°C. Src was immunoprecipitated and immunoblotted with antibodies against Src, anti-pY416^{src} antibodies or antibodies against PK2.

The same lysates were mixed with a GST fusion protein containing the SH2 domain of Src and the bound proteins were analyzed by immunoblotting with antibodies against PYK2.

293T cells were transiently transfected with pRK5, PYK2, PYK2-Y402F and PKM. Total cell lysates were analyzed by immunoblotting with anti-pTyr or anti-PYK2 antibodies. The same lysates were subjected to immunoprecipitation with antibodies against *src* and immunoblotting with anti-pY416^{src} or antibodies against Src. Src kinase activity was quantitated as an increase in pY416^{src} phosphorylation and presented as mean +/-S.D. from four independent experiments.

The effects of coexpression of PYK2 and Csk on PYK2 tyrosine phosphorylation and MAP kinase activation were also assessed. pRK5, PYK2, PYK2-Y402F or PYK2 plus increasing concentrations of Csk were transiently transfected in 293T cells. Total cell lysates were analyzed by immunoblotting with antibodies against phosphotyrosine, PYK2 and Csk. A weak tyrosine phosphorylation of PYK2 Y402F was observed upon longer exposure. The same lysates were used to determine MAP kinase activation. This experiment was repeated three times. It was thus determined that PKY2 and Src are involved in MAP kinase activation.

### Example 15: PYK2 and Src Activate MAPK

We further examined the status of Src phosphorylation and MAP kinase activation upon transfection of PYK2 or PYK2-Y402F in human embryonic kidney 293T cells. Overexpression of PYK2 leads to approximately four fold stimulation of endogenous Src activity in these cells. However, overexpression of PYK2-Y402F or a kinase negative mutant of PYK2 (PKM) did not affect Src activity in the same assay. These experiments demonstrated that autophosphorylation of PYK2 on Tyr402 leads to the binding of the SH2 domain of Src and subsequent Src activation.

We next overexpressed PYK2, PYK2-Y402F or PKM with increasing amounts of Csk, a protein tyrosine kinase that negatively regulates Src (Nada *et al., Nature* 351: 69-72 (1991), and tested whether PYK2-induced Src activation contributes to PYK2 tyrosine phosphorylation and PYK2-induced MAP kinase activation in 293T cells. The tyrosine phosphorylation of PYK2 and PYK2-induced MAP kinase activition, normally observed from cells overexpressing PYK2, were significantly reduced in the presence of increasing amounts of Csk. Lev *et al., Nature* 376: 737-745 (1995). In addition, MAP kinase activation was greatly reduced in cells overexpressing PYK2-Y402F as compared to MAP kinase activation induced by expression of wild-type PYK2. PYK2-Y402F is poorly tyrosine phosphorylated upon overexpression. Moreover overexpression of PYK2-Y402F did not activate Src in these experiments. These experiments demonstrate that PYK2 tyrosine phosphorylation and PYK2-induced MAP kinase activation are dependent, at least in part, on Src kinase activity stimulated by binding to autophosphorylated tyr402 on PYK2.

We next examined the role of PYK2 in LPA- or bradykinin-induced MAP kinase activation by utilizing the dominant interfering kinase negative mutant of PYK2. Lev *et al, Nature* 376:737-745 (1995); Tokiwa *et al., Science* 273: 792-794 (1996). We were unable to generate stable PC12 cell lines that overexpress PKM and therefore used a transient overexpression strategy. Overexpression of PKM in PC12 cells strongly inhibited MAP kinase activation by LPA or bradykinin. The role of Src in LPA- or bradykinin-induced MAP kinase activation was further tested by transient overexpression of Csk. When Csk was overexpressed in PC12 cells a strong reduction in LPA- or bradykinin-induced MAP kinase was observed. In cells that were co-transfected with both PKM and Csk, LPA- or bradykinin-induced MAP kinase activation was profoundly inhibited. However, overexpression of PKM or Csk did not affect EGF- or nerve growth factor-induced MAP kinase activation in PC12.

Taken together, these experiments reveal a specific role for PYK2and Src in linking G-protein coupled receptor, but not growth factor receptors, with MAP kinase activation.

## Claims

1. A method of screening for a compound capable of binding to a PYK2 polypeptide, wherein said compound is an indolinone, comprising the steps of:
(a) incubating said compound with said PYK2 polypeptide, and
(b) detecting the presence of said compound bound to said PYK2 polypeptide.

2. The method according to claim 1, wherein said compound inhibits a phosphorylation activity of said PYK2 polypeptide.

3. A method of screening potential agents useful for treatment of a disease or condition **characterised by** an abnormality in a signal transduction pathway, wherein said signal transduction pathway includes an interaction between a PYK2 polypeptide and a natural binding partner, comprising the step of assaying said potential agents for those able to promote or disrupt said interaction as an indication of a useful agent, wherein said natural binding partner is Src, and wherein said pathway involves a G protein-coupled receptor but does not involve a growth factor receptor, and wherein said potential agents are one or more indolinones.

4. The method of claim 3, wherein said indolinone is an oxindolinone.

5. A method of screening for modulators of PYK2 signaling pathways comprising the steps of:
(a) mixing two or more components selected from the group consisting of PYK2, Src, bradykinin, LPA, and one or more of said modulators wherein said modulators are one or more indolinones; and
(b) monitoring the effect of said modulators on said PYK2 signaling pathways.

6. The method of claim 5 wherein said indolinone is an oxindolinone.

7. The method of claim 5 or claim 6 wherein said method involves screening agents capable of promoting or disrupting the binding of the SH2 domain of Src to the tyrosine at position 402 of PYK2.

8. The method of any of claims 5 to 7 wherein said effect is a phosphorylation state of PYK2 or Src.

## Patentansprüche

1. Screeningverfahren für eine Verbindung, die eine Bindung mit einem PYK2-Polypeptid eingehen kann, wobei die genannte Verbindung ein Indolinon ist, wobei das Verfahren die folgenden Schritte umfasst:
(a) Inkubieren der genannten Verbindung mit dem genannten PYK2-Polypeptid und
(b) Detektieren des Vorhandenseins der genannten Verbindung, die mit dem genannten PYK2-Polypeptid gebunden ist.

2. Verfahren nach Anspruch 1, wobei die genannte Verbindung eine Phosphorylierungsaktivität des genannten PYK2-Polypeptids verhindert.

3. Verfahren zum Screening potenziell nützlicher Mittel zur Behandlung einer Erkrankung oder eines Zustand, die bzw. der durch eine Anomalität in einem Signaltransduktionspfad **gekennzeichnet** ist, wobei der genannte Signaltransduktionspfad eine Interaktion zwischen einem PYK2-Polypeptid und einem natürlichen Bindungspartner aufweist, wobei das Verfahren als Indikation für ein nützliches Mittel den Schritt der Auswertung der genannten potenziellen Mittel in die Mittel umfasst, die in der Lage sind, die genannte Interaktion zu fördern oder zu stören, wobei der genannten natürliche Bindungspartner Src ist, und wobei der genannte Pfad einen G-Protein gekoppelten Rezeptor aufweist, jedoch keinen Wachstumsfaktorrezeptor, und wobei es sich bei den genannten potenziellen Mitteln um ein oder mehrere Indolinone handelt.

4. Verfahren nach Anspruch 3, wobei es sich bei dem genannten Indolinon um ein Oxindolinon handelt.

5. Verfahren zum Screening nach Modulatoren von PYK2-Signalisierungspfaden, wobei das Verfahren die folgenden Schritte umfasst:
(a) Mischen von zwei oder mehr Komponenten, die aus der Gruppe ausgewählt werden, die PYK2, Src, Bradykinin, LPA umfasst, und eines oder mehrerer der genannten Modulatoren, wobei es sich bei den genannten Modulatoren um ein oder mehrere Indolinone handelt; und
(b) Überwachen des Effekts der genannten Modulatoren auf die genannten PYK2-Signalisierungspfade.

6. Verfahren nach Anspruch 5, wobei es sich bei dem genannten Indolinon um ein Oxindolinon handelt.

7. Verfahren nach Anspruch 5 oder 6, wobei das genannte Verfahren das Screening von Mitteln umfasst, welche die Bindung der SH2-Domäne von Src mit dem Tyrosin an der Position 402 von PYK2 fördern oder behindern können.

8. Verfahren nach einem der Ansprüche 5 bis 7, wobei es sich bei dem genannten Effekt um einen Phosphorylierungszustand von PYK2 oder Src handelt.

## Revendications

1. Un procédé de dépistage d'un composé capable de se lier au polypeptide PYK2, où le ledit composé est une indolinone, qui comprend les étapes suivantes :
Incubation dudit composé avec ledit polypeptide PYK2.
Détection de la présence dudit composé lié audit polypeptide PYK2.

2. Le procédé conforme à la revendication 1, où ledit composé inhibe l'activité de phosphorylation dudit polypeptide PYK2.

3. Un procédé de dépistage des agents potentiellement utiles dans le traitement de maladies ou de conditions **caractérisées par** une anormalité au niveau de la voie de transduction du signal, où ladite voie de transduction du signal comprend une interaction entre le polypeptide PYK2 et un composé naturel avec lequel il entre en liaison ; incluant une étape de sélection desdits agents potentiels capables de promouvoir ou déranger ladite interaction comme étant une caractéristique dudit agent, où ledit partenaire de liaison naturel est une Src, et où ladite voie comprend un récepteur couplé à la protéine G mais sans inclure de récepteur du facteur de croissance. et où lesdits agents potentiels sont une ou plusieurs indolinones.

4. Le procédé de la revendication 3, où ladite indolinone est une oxindolinone.

5. Un procédé de dépistage des modulateurs des voies de signal de PYK2 comprenant les étapes :
Mélange d'au moins deux composants sélectionnés dans un groupe comprenant PYK2, Src, brakinine, LPA et un ou plus desdits modulateurs où lesdits modulateurs sont une ou plusieurs indolinones ; et
Contrôle des effets desdits modulateurs sur les voies de signal de ladite PYK2.

6. Le procédé de la revendication 5 où ladite indolinone est une oxindolinone.

7. Le procédé de la revendication 5 ou de la revendication 6 où ledit procédé comprend un dépistage des agents capables de promouvoir ou déranger la liaison du domaine SH2 de Src à la Tyrosine à la position 402 de PYK2.

8. Le procédé des revendications 5 ou 7 où ledit effet est un état de phosphorylation de PYK2 ou Src.


### SEQUENCE LISTING

(1) GENERAL INFORMATION:
   (i) APPLICANT:
   (ii) TITLE OF INVENTION: PYK2 RELATED PRODUCTS AND METHODS
   (iii) NUMBER OF SEQUENCES: 24
   (iv) CORRESPONDENCE ADDRESS:
      (A) ADDRESSEE: Lyon & Lyon
      (B) STREET: 633 West Fifth Street
      (C) CITY: Los Angeles
      (D) STATE: California
      (E) COUNTRY: USA
      (F) ZIP: 90071
   (v) COMPUTER READABLE FORM:
      (A) MEDIUM TYPE: 3.5" Diskette, 1.44 Mb storage
      (B) COMPUTER: IBM Compatible
      (C) OPERATING SYSTEM: IBM P.C. DOS (Version 5.0)
      (D) SOFTWARE: WordFerfect (Version 5.1)
   (vi) CURRENT APPLICATION DATA:
      (A) APPLICATION NUMBER:
      (B) FILING DATE:
      (C) CLASSIFICATION:
      (vii) PRIOR APPLICATION DATA:
      (A) APPLICATION NUMBER:
      (B) FILING DATE:
   (viii) ATTORNEY/AGENT INFORMATION:
      (A) NAME: Warburg, Richard J.
      (B) REGISTRATION NUMBER: 32,327
      (C) REFERENCE/DOCKET NUMBER: 211/121
   (ix) TELECOMMUNICATION INFORMATION:
      (A) TELEPHONE: (213) 489-1600
      (B) TELEFAX: (213) 955-0440
      (C) TELEX: 67-3510
(2) INFORMATION FOR SEQUENCE ID NO: 1:
   (i) SEQUENCE CHARACTERISTICS:
      (A) LENGTH: 1009
      (B) TYPE: amino acid
      (C) STRANDNESS: single
      (D) TOPOLOGY: linear
   (ii) MOLECULE TYPE: peptide
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 3416
      (B) TYPE: nucleic acid
      (C) STRANDNESS: single
      (D) TOPOLOGY: linear
   (ii) MOLECULE TYPE: nucleic
   (xi) SEQUENCE DESCRIPTION: SEQ ID NO: 2:
   CGGTACAGGT AAGTCGGCCG GGCAGGTAGG GGTGCCCGAG GAGTAGTCGC TGGAGTCCGC    60
   GCCTCCCTGG GACTGCAATG TGCCGGTCTT AGCTGCTGCC TGAGAGGATG TCTGGGGTGT   120
   CCGAGCCCCT GAGCCGAGTA AAGTTGGGCA CATTACGCCG GCCTGAAGGC CCTGCAGAGC   180
   CCATGGTGGT GGTACCAGTA GATGTGGAAA AGGAGGACGT GCGTATCCTC AAGGTCTGCT   240
   TCTATAGCAA CAGCTTCAAT CCTGGGAAGA ACTTCAAACT GGTCAAATGC ACTGTCCAGA   300
   CGGAGATCCG GGAGATCATC ACCTCCATCC TGCTGAGCGG GCGGATCGGG CCCAACATCC   360
   GGTTGGCTGA GTGCTATGGG CTGAGGCTGA AGCACATGAA GTCCGATGAG ATCCACTGGC   420
   TGCACCCACA GATGACGGTG GGTGAGGTGC AGGACAAGTA TGAGTGTCTG CACGTGGAAG   480
   CCGAGTGGAG GTATGACCTT CAAATCCGCT ACTTGCCAGA AGACTTCATG GAGAGCCTGA   540
   AGGAGGACAG GACCACGCTG CTCTATTTTT ACCAACAGCT CCGGAACGAC TACATGCAGC   600
   GCTACGCCAG CAAGGTCAGC GAGGGCATGG CCCTGCAGCT GGGCTGCCTG GAGCTCAGGC   660
   GGTTCTTCAA GGATATGCCC CACAATGCAC TTGACAAGAA GTCCAACTTC GAGCTCCTAG   720
   AAAAGGAAGT GGGGCTGGAC TTGTTTTTCC CAAAGCAGAT GCAGGAGAAC TTAAAGCCCA   780
   AACAGTTCCG GAAGATGATC CAGCAGACCT TCCAGCAGTA CGCCTCGCTC AGGGAGGAGG   840
   AGTGCGTCAT GAAGTTCTTC AACACTCTCG CCGGCTTCGC CAACATCGAC CAGGAGACCT 900
   ACCGCTGTGA ACTCATTCAA GGATGGAACA TTACTGTGGA CCTGGTCATT GGCCCTAAAG 960
   GGATCCGCCA GCTGACTAGT CAGGACGCAA AGCCCACCTG CCTGGCCGAG TTCAAGCAGA 1020
   TCAGGTCCAT CAGGTGCCTC CCGCTGGAGG AGGGCCAGGC AGTACTTCAG CTGGGCATTG 1080
   AAGGTGCCCC CCAGGCCTTG TCCATCAAAA CCTCATCCCT AGCAGAGGCT GAGAACATGG 1140
   CTGACCTCAT AGACGGCTAC TGCCGGCTGC AGGGTGAGCA CCAAGGCTCT CTCATCATCC 1200
   ATCCTAGGAA AGATGGTGAG AAGCGGAACA GCCTGCCCCA GATCCCCATG CTAAACCTGG 1260
   AGGCCCGGCG GTCCCACCTC TCAGAGAGCT GCAGCATAGA GTCAGACATC TACGCAGAGA 1320
   TTCCCGACGA AACCCTGCGA AGGCCCGGAG GTCCACAGTA TGGCATTGCC CGTGAAGATG 1380
   TGGTCCTGAA TCGTATTCTT GGGGAAGGCT TTTTTGGGGA GGTCTATGAA GGTGTCTACA 1440
   CAAATCACAA AGGGGAGAAA ATCAATGTAG CTGTCAAGAC CTGCAAGAAA GACTGCACTC 1500
   TGGACAACAA GGAGAAGTTC ATGAGCGAGG CAGTGATCAT GAAGAACCTC GACCACCCGC 1560
   ACATCGTGAA GCTGATCGGC ATCATTGAAG AGGAGCCCAC CTGGATCATC ATGGAATTGT 1620
   ATCCCTATGG GGAGCTGGGC CACTACCTGG AGCGGAACAA GAACTCCCTG AAGGTGCTCA 1680
   CCCTCGTGCT GTACTCACTG CAGATATGCA AAGCCATGGC CTACCTGGAG AGCATCAACT 1740
   GCGTGCACAG GGACATTGCT GTCCGGAACA TCCTGGTGGC CTCCCCTGAG TGTGTGAAGC 1800
   TGGGGGACTT TGGTCTTTCC CGGTACATTG AGGACGAGGA CTATTACAAA GCCTCTGTGA 1860
   CTCGTCTCCC CATCAAATGG ATGTCCCCAG AGTCCATTAA CTTCCGACGC TTCACGACAG 1920
   CCAGTGACGT CTGGATGTTC GCCGTGTGCA TGTGGGAGAT CCTGAGCTTT GGGAAGCAGC 1980
   CCTTCTTCTG GCTGGAGAAC AAGGATGTCA TCGGGGTGCT GGAGAAAGGA GACCGGCTGC 2040
   CCAAGCCTGA TCTCTGTCCA CCGGTCCTTT ATACCCTCAT GACCCGCTGC TGGGACTACG 2100
   ACCCCAGTGA CCGGCCCCGC TTCACCGAGC TGGTGTGCAG CCTCAGTGAC GTTTATCAGA 2160
   TGGAGAAGGA CATTGCCATG GAGCAAGAGA GGAATGCTCG CTACCGAACC CCCAAAATCT 2220
   TGGAGCCCAC AGCCTTCCAG GAACCCCCAC CCAAGCCCAG CCGACCTAAG TACAGACCCC 2280
   CTCCGCAAAC CAACCTCCTG GCTCCAAAGC TGCAGTTCCA GGTTCCTGAG GGTCTGTGTG 2340
   CCAGCTCTCC TACGCTCACC AGCCCTATGG AGTATCCATC TCCCGTTAAC TCACTGCACA 2400
   CCCCACCTCT CCACCGGCAC AATGTCTTCA AACGCCACAG CATGCGGGAG GAGGACTTCA 2460
   TCCAACCCAG CAGCCGAGAA GAGGCCCAGC AGCTGTGGGA GGCTGAAAAG GTCAAAATGC 2520
   GGCAAATCCT GGACAAACAG CAGAAGCAGA TGGTGGAGGA CTACCAGTGG CTCAGGCAGG 2580
   AGGAGAAGTC CCTGGACCCC ATGGTTTATA TGAATGATAA GTCCCCATTG ACGCCAGAGA 2640
   AGGAGGTCGG CTACCTGGAG TTCACAGGGC CCCCACAGAA GCCCCCGAGG CTGGGCGCAC 2700
   AGTCCATCCA GCCCACAGCT AACCTGGACC GGACCGATGA CCTGGTGTAC CTCAATGTCA 2760
   TGGAGCTGGT GCGGGCCGTG CTGGAGCTCA AGAATGAGCT CTGTCAGCTG CCCCCCGAGG 2820
   GCTACGTGGT GGTGGTGAAG AATGTGGGGC TGACCCTGCG GAAGCTCATC GGGAGCGTGG 2880
   ATGATCTCCT GCCTTCCTTG CCGTCATCTT CACGGACAGA GATCGAGGGC ACCCAGAAAC 2940
   TGCTCAACAA AGACCTGGCA GAGCTCATCA ACAAGATGCG GCTGGCGCAG CAGAACGCCG 3000
   TGACCTCCCT GAGTGAGGAG TGCAAGAGGC AGATGCTGAC GGCTTCACAC ACCCTGGCTG 3060
   TGGACGCCAA GAACCTGCTC GACGCTGTGG ACCAGGCCAA GGTTCTGGCC AATCTGGCCC 3120
   ACCCACCTGC AGAGTGACGG AGGGTGGGGG CCACCTGCCT GCGTCTTCCG CCCCTGCCTG 3180
   CCATGTACCT CCCCTGCCTT GCTGTTGGTC ATGTGGGTCT TCCAGGGAGA AGGCCAAGGG 3240
   GAGTCACCTT CCCTTGCCAC TTTGCACGAC GCCCTCTCCC CACCCCTACC CCTGGCTGTA 3300
   CTGCTCAGGC TGCAGCTGGA CAGAGGGGAC TCTGGGCTAT GGACACAGGG TGACGGTGAC 3360
   AAAGATGGCT CAGAGGGGGA CTGCTGCTGC CTGGCCACTG CTCCCTAAGC CAGCCT 3416
(2) INFORMATION FOR SEQ ID NO: 3:
   (i) SEQUENCE CHARACTERISTICS:
      (A) LENGTH: 10
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
(2) INFORMATION FOR SEQ ID NO: 4:
   (i) SEQUENCE CHARACTERISTICS:
      (A) LENGTH: 8
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 4:
   Trp Met Phe Gly Val Thr Leu Trp
                   5
(2) INFORMATION FOR SEQ ID NO: 5:
   (i) SEQUENCE CHARACTERISTICS:
      (A) LENGTH: 30
      (B) TYPE: nucleic acid
      (C) STRANDEDNESS: single
      (D) TOPOLOGY: linear
   (ii) SEQUENCE DESCRIPTION: SEQ ID NO: 5:
   CGGGATCCTC ATCATCCATC CTAGGAAAGA          30
(2) INFORMATION FOR SEQ ID NO: 6:
   (i) SEQUENCE CHARACTERISTICS:
      (A) LENGTH: 30
      (B) TYPE: nucleic acid
      (C) STRANDEDNESS: single
      (D) TOPOLOGY: linear
   (ii) SEQUENCE DESCRIPTION: SEQ ID NO: 6:
   CGGGAATTCG TCGTAGTCCC AGCAGCGGGT          30
(2) INFORMATION FOR SEQ ID NO: 7:
   (i) SEQUENCE CHARACTERISTICS:
      (A) LENGTH: 10
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 7:
   Tyr Pro Tyr Asp Val Pro Asp Tyr Ala Ser
                    5                   10
(2) INFORMATION FOR SEQ ID NO: 8:
   (i) SEQUENCE CHARACTERISTICS:
      (A) LENGTH: 11
      (B) TYPE: nucleic acid
      (C) STRANDEDNESS: single
      (D) TOPOLOGY: linear
   (ii) SEQUENCE DESCRIPTION: SEQ ID NO: 8:
(2) INFORMATION FOR SEQ ID NO: 9:
   (i) SEQUENCE CHARACTERISTICS:
      (A) LENGTH: 63
      (B) TYPE: nucleic acid
      (C) STRANDEDNESS: single
      (D) TOPOLOGY: linear
   (ii) SEQUENCE DESCRIPTION: SEQ ID NO: 9:
   GGCTCTAGAT CACGATGCGT AGTCAGGGAC ATCGTATGGG TACTCTGCAG GTGGGTGGGC 60
   CAG 63
(2) INFORMATION FOR SEQ ID NO: 10:
   (i) SEQUENCE CHARACTERISTICS:
      (A) LENGTH: 31
      (B) TYPE: nucleic acid
      (C) STRANDEDNESS: single
      (D) TOPOLOGY: linear
   (ii) SEQUENCE DESCRIPTION: SEQ ID NO: 10:
   CAATGTAGCT GTCGCGACCT GCAAGAAAGA C 31
(2) INFORMATION FOR SEQ ID NO: 11:
   (i) SEQUENCE CHARACTERISTICS:
      (A) LENGTH: 11
      (B) TYPE: nucleic acid
      (C) STRANDEDNESS: single
      (D) TOPOLOGY: linear
   (ii) SEQUENCE DESCRIPTION: SEQ ID NO: 11:
(2) INFORMATION FOR SEQ ID NO: 12:
   (i) SEQUENCE CHARACTERISTICS:
      (A) LENGTH: 60
      (B) TYPE: nucleic acid
      (C) STRANDEDNESS: single
      (D) TOPOLOGY: linear
   (ii) SEQUENCE DESCRIPTION: SEQ ID NO: 12:
   CGGAATTCTT ACGATGCGTA GTCAGGGACA TCGTATGGGT AGACATCAGT TAACATTTTG 60
(2) INFORMATION FOR SEQ ID NO: 13:
   (i) SEQUENCE CHARACTERISTICS:
      (A) LENGTH: 9
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 13:
   Ile His Arg Asp Leu Ala Ala Arg Asn
   5
(2) INFORMATION FOR SEQ ID NO: 14:
   (i) SEQUENCE CHARACTERISTICS:
      (A) LENGTH: 5
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 14:
(2) INFORMATION FOR SEQ ID NO: 15:
   (i) SEQUENCE CHARACTERISTICS:
      (A) LENGTH: 4
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 15:
   Tyr Leu Met Val
(2) INFORMATION FOR SEQ ID NO: 16:
   (i) SEQUENCE CHARACTERISTICS:
      (A) LENGTH: 4
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 16:
   Tyr Val Val Val
(2) INFORMATION FOR SEQ ID NO: 17:
   (i) SEQUENCE CHARACTERISTICS:
      (A) LENGTH: 9
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 17:
   Ile His Arg Asp Leu Ala Ala Arg Asn
                   5
(2) INFORMATION FOR SEQ ID NO: 18:
   (i) SEQUENCE CHARACTERISTICS:
      (A) LENGTH: 5
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 18:
(2) INFORMATION FOR SEQ ID NO: 19:
   (i) SEQUENCE CHARACTERISTICS:
      (A) LENGTH: 10
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 19:
   Tyr Pro Tyr Asp Val Pro Asp Tyr Ala Ser
                   5                   10
(2) INFORMATION FOR SEQ ID NO: 20:
   (i) SEQUENCE CHARACTERISTICS:
      (A) LENGTH: 11
      (B) TYPE: nucleic acid
      (C) STRANDEDNESS: single
      (D) TOPOLOGY: linear
   (ii) SEQUENCE DESCRIPTION: SEQ ID NO: 20:
(2) INFORMATION FOR SEQ ID NO: 21:
   (i) SEQUENCE CHARACTERISTICS:
      (A) LENGTH: 63
      (B) TYPE: nucleic acid
      (C) STRANDEDNESS: single
      (D) TOPOLOGY: linear
   (ii) SEQUENCE DESCRIPTION: SEQ ID NO: 21:
   GGCTCTAGAT CACGATGCGT AGTCAGGGAC ATCGTATGGG TACTCTGCAG GTGGGTGGGC   60
   CAG                                                                 63
(2) INFORMATION FOR SEQ ID NO: 22:
   (i) SEQUENCE CHARACTERISTICS:
      (A) LENGTH: 10
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 22:
   Tyr Pro Tyr Asp Val Pro Asp Tyr Ala Ser
                       5                  10
(2) INFORMATION FOR SEQ ID NO: 23:
   (i) SEQUENCE CHARACTERISTICS:
      (A) LENGTH: 11
      (B) TYPE: nucleic acid
      (C) STRANDEDNESS: single
      (D) TOPOLOGY: linear
   (ii) SEQUENCE DESCRIPTION: SEQ ID NO: 23:
(2) INFORMATION FOR SEQ ID NO: 24:
   (i) SEQUENCE CHARACTERISTICS:
      (A) LENGTH: 60
      (B) TYPE: nucleic acid
      (C) STRANDEDNESS: single
      (D) TOPOLOGY: linear
   (ii) SEQUENCE DESCRIPTION: SEQ ID NO: 24:
   CGGAATTCTT ACGATGCGTA GTCAGGGACA TCGTATGGGT AGACATCAGT TAACATTTTG    60
(2) INFORMATION FOR SEQ ID NO: 25:
   (i) SEQUENCE CHARACTERISTICS:
      (A) LENGTH: 31
      (B) TYPE: nucleic acid
      (C) STRANDEDNESS: single
      (D) TOPOLOGY: linear
   (ii) SEQUENCE DESCRIPTION: SEQ ID NO: 25:
   CAATGTAGCT GTCGCGACCT GCAAGAAAGA C          31